(19) Europäisches Patentamt
European Patent Office
Office européen des brevets

(11) Publication number: 0 223 417
A1

## (12) EUROPEAN PATENT APPLICATION

(21) Application number: 86308064.4

(22) Date of filing: 17.10.86

(51) Int. Cl.⁴: **C12N 15/00** , C07H 21/04 , C12N 5/00 , A01H 1/00 , C12P 19/34 , //(C12N15/00,C12R1:19)

The applicant has filed a statement in accordance with Rule 28 (4) EPC (issue of a sample only to an expert).

(30) Priority: 21.10.85 US 788984

(43) Date of publication of application: 27.05.87 Bulletin 87/22

(84) Designated Contracting States: AT BE CH DE ES FR GB GR IT LI LU NL SE

(71) Applicant: **LUBRIZOL GENETICS INC. 3375 Mitchell Lane Boulder Colorado 80301-2244(US)**

(72) Inventor: **Sutton, Dennis W. 5214 Kevins Way Madison Wisconsin 53714(US)**
Inventor: **Merlo, Donald J. 816 Creed Las Cruces New Mexico, 88005(US)**
Inventor: **Talbot, Duncan R. 306 Quail Village Court Ballwin Missouri 63021(US)**

(74) Representative: **Fisher, Adrian John et al Carpmaels & Ransford 43 Bloomsbury Square London WC1A 2RA(GB)**

(54) TL-based sub-T-DNA plasmids.

(57) Sub-Ti plasmids based on $T_L$ -DNA from an octopine-type Ti plasmid are disclosed. Selectable markers funtional in eukaryotes are also exemplified, as are vectors and methods useful in efforts to transform plants.

Figure 2

## T$_L$-BASED SUB-T-DNA PLASMIDS

### FIELD

The present invention is in the fields of genetics engineering and plant husbandry, and especially provides vectors useful for plant transformation and means for promotion of transcript termination in plants.

### BACKGROUND

Following are publications which disclose background information closely related to the present invention. Octopine T$_L$-DNA-based sub-Ti plasmids are disclosed by Deblaere R et al. (1985) Nucl. Acids Res. 13:4777-4788; Klee HJ et al. (1985) Biotechnol. 3:637-642; Dahl GA et al., European Patent application 140,556; Hoekema A (1985) Ph.D. Thesis, Rijksuniversiteit Leiden, The Netherlands; and Barker RF et al. - (1983) Plant Mol. Biol. 2:335-350 (see the Background section on Genes on the Transformation-Inducing Plasmids). Fraley RT et al. (1985) Biotechnol. 3 :629-635, discloses a split end vector system. Use of CaMV and ORF24 promoters to drive expression of NPT2 structural genes are disclosed by Koziel MG et al. - (1985) J. Mol. Appl. Genet. 2 :549-562, and Gelvin SB et al. (1985) Mol. Gen. Genet. 199:240-248 - (Selectable or Screenable Markers).

#### Overview of Agrobacterium

Virulent strains of the gram-negative genus Agrobacterium harbor large plasmids known as Ti (tumor-inducing or transformation-inducing) plasmids (pTi) in A. tumefaciens and Ri (root-inducing) plasmids (pRi) in A. rhizogenes, often classified by the opine which they catabolize or cause to be synthesized. Ti and Ri plasmids both contain DNA sequences, known as T-DNA (transferred-DNA), which in tumors are found to be integrated into the genome of the host plant. Several T-DNA genes are under control of T-DNA promoters which resemble canonical eukaryotic promoters in structure. The Ti plasmid also carries genes outside the T-DNA region. Ti and Ri plasmids are for many purposes functionally equivalent.

Reviews of Agrobacterium-caused disease, plant transformation, genetic engineering, and gene expression include those by, or found in, Merlo DJ (1982) Adv. Plant Pathol. 1:139-178; Ream LW and Gordon MP (1982), Science 218:854-859; Bevan MW and Chilton M-D (1982), Ann. Rev. Genet. 16:357-384; Kahl G and Schell J (1982) Molecular Biology of Plant Tumors; Barton KA and Chilton M-D (1983) Meth. Enzymol. 101 :527-539; Depicker A et al. (1983) in Genetic Engineering of Plants: an Agricultural Perspective, eds: Kosuge T et al., pp. 143-176; Caplan A et al. (1983) Science 222:815-821; Hall TC et al., European Patent application 126,546; Binns AN (1984) Oxford Surveys Plant Mol. Cell Biol. 1:130-160; Hall TC (1985) Oxford Surveys Plant Mol. Biol. 2:329-338; Hooykaas PJJ and Schilperoort RA (1985) Trends Biochem. Sci. 10:307-309; Pühler A, ed. (1983) Molecular Genetics of the Bacteria-Plant Interaction; and Schilperoort RA - (1984) in Efficiency in Plant Breeding (Proc. 10th Congr. Eur. Assoc. Res. Plant Breeding), eds: Lange W et al., pp. 251-285.

#### Infection of Plant Tissues

Plant cells can be transformed by Agrobacterium by several methods known in the art. For a review of recent work, see Syono K (1984) Oxford Surveys Plant Mol. Cell Biol. 1:217-219.

The infection of plant tissue by Agrobacterium is a simple technique well known to those skilled in the art. Typically after being wounded, a plant is inoculated with a suspension of the bacteria. Alternatively, tissue pieces are innoculated, e.g. leaf disks (Horsch RB et al. (1985) Science 227:1229-1231), or inverted stem segments (Barton KA et al. (1983) Cell 32:1033-1043). Traditional inoculation and culture techniques may be modified for use of disarmed T-DNA vectors incapable of hormone-independent growth (e.g. see Zambryski P et al . (1984) in Genetic Engineering, Principles, and Methods, 6, eds.: Hollaender A and Setlow J).

Agrobacterium is also capable of infecting isolated cells, cells grown in culture, callus cells, and isolated protoplasts (e.g. Horsch RB and Fraley RT (1983) in Advances in Gene Technology: Molecular Genetics of Plants and Animals (Miami Winter Symposium 20), eds.: Downey K et al., p. 576; Fraley RT et al. (1984) Plant Mol. Biol. 3:371-378; Fraley RT and Horsch RB (1983) in Genetic Engineering of Plants: an Agricultural Perspective, eds.: Kosuge T et al., pp. 177-194; Muller A et al. (1983) Biochem. Biophys. Res. Comm. 123:458-462). The transformation frequency of inoculated callus pieces can be increased by addition of an opine or opine precursors (Cello LM and Olsen WL, U.S. Patent 4,459,355).

The host range of crown gall pathogenesis may be influenced by T-DNA-encoded functions such as onc genes (Hoekema A et al . (1984) J. Bacteriol. 158:383-385; Hoekema A et al. (1984) EMBO J. 3:3043-3047; Buchholz WC and Thomasshow MF (1984) 160 :327-332). Ausich RL, European Patent Application 108,580, reports transfer of T-DNA from A. tumefaciens to green algal cells, and expression therein of octopine synthase and Tn5 kanamycin resistance genes. Hooykaas-van Slogteren GMS et al. (1984) Nature 311:763-764, and Hernalsteens J-P et al. (1984) EMBO J. 3:3039-3041, have demonstrated transformation of monocot cells by Agrobacterium without the customary tumorigenesis.

Regeneration of Plants

Genes involved in opine anabolism were capable of passing through meiosis, though the plants were male sterile if the T-DNA was not disarmed. T-DNA, disarmed T-DNA, and functional foreign genes can be inherited seemingly unaltered in a dominant, closely-linked, Mendelian fashion (e.g. see Horsch RB et al. - (1984) Science 223:496-498; Tepfer D (1984) Cell 37:959-967; DeBlock M et al. (1984) EMBO J. 3:1681-1689; Wöstemeyer A et al. (1984) Mol. Gen. Genet. 194:500-507.

Plant cells transformed by totally disarmed T-DNA can usually be regenerated into whole plants by using regeneration protocols effective for regeneration of untransformed plants of that particular species. The epigenetic state of the plant cells initially transformed can affect regeneration potential (van Slogteren GMS et al. (1983) Plant Mol. Biol. 2:321-333).

Genes on the Transformation-Inducing Plasmids

The complete sequence of the T-DNA of an octopine-type plasmid found in ATCC 15955, pTi15955,has been reported (Barker RF et al. (1983) Plant Mol. Biol. 2:335-350) as has the $T_L$ region of pTiAch5 (Gielen J et al . (1984) EMBO J. 3:835-846). Published T-DNA genes do not contain introns. Sequences resembling canonical eukaryotic promoter elements and polyadenylation sites can be recognized.

A number of genes have been identified within the T-DNA of the Ti or Ri plasmids. Several plasmid T-DNA transcripts have been mapped (e.g. Willmitzer L et al. (1982) EMBO J. 1:139-146; Karcher SJ et al. - (1984) Mol. Gen. Genet. 194:159-165 and some functions have been assigned. Octopine Ti plasmids having mutations in the genes tms, tmr, tml , ons, and ocs respectively incite tumorous calli of Nicotiana tabacum which generate shoots, proliferate roots, are larger than normal, do not secrete opines, and do not synthesize octopine; tms, tmr, and tml are onc (oncogenicity) genes. In other hosts, mutants of these genes can induce different phenotypes (see Bevan and Chilton, Ann. Rev. Genet., supra). Mutations in T-DNA genes do not affect insertion of T-DNA into the plant genome (Leemans J etal. (1982) EMBO J. 1:147-152; Ream LW et al. (1983) Proc. Natl. Acad. Sci. USA 80:1660-1664).

Octopine Ti plasmids carry an ocs gene which encodes octopine synthase (lysopine dehydrogenase). Koncz C et al. (1983) EMBO J. 2:1597-1603 provides a functional analysis of ocs. Dhaese P. et al. (1983) EMBO J. 2:419-426, reported the utilization of various polyadenylation sites by "transcript 7" (ORF3 of Barker R et al., supra) and ocs. The presence of the enzyme octopine synthase within a tissue can protect that tissue from the toxic effect of various amino acid analogs (Dahl GA and Tempé J, (1983) Theor. Appl. Genet. 66:233-239; Koziel MG et al. (1984) J. Mol. Appl. Genet. 2 :549-562).

Nopaline Ti plasmids encode the nopaline synthase gene (nos) (sequenced by Depicker A et al. (1982) J. Mol. Appl. Genet. 1:561-573). Shaw CH et al. (1984) Nucl. Acids Res. 12 :7831-7846, provides a functional analysis of nos.

Ti and Ri plasmid genes outside of the T-DNA region include the vir genes, which when mutated result in an avirulent Ti plasmid. Several vir genes have been accurately mapped and have been found to be located in regions conserved among various Ti plasmids (Iyer VN et al. (1982) Mol. Gen. Genet. 188:418-424). Vir genes of different Ti plasmids can promote transformation at different levels (Otten L et al. (1985) Mol. Gen. Genet.199:189-193). The vir genes function in trans, being capable of causing the transformation of plant cells with T-DNA of a different plasmid type and physically located on another plasmid (e.g. Deblaere R et al. (1985) Nulc. Acids Res. 13:4777-4788; de Framond AJ et al. (1983) Biotechnol. 1:262-269; Hoekema A et al. (1983) Nature 303:179-180; Hille J et al. (1984) J. Bacteriol. 158:754-756; Hoekema A et al . (1984) J. Bacteriol. 158:383-385; An G et al. (1985) EMBO J. 4:277-284; Klee HJ et al. (1985) Biotechnol. 3:637-642); such arrangements are known as binary systems. Chilton et al. (18 January 1983) 15th Miami Winter Symp., described a "micro-Ti" plasmid made by resectioning the mini-Ti of de Framond et al., supra, (see European patent application 126,546 for a description). Dahl GA et al., European Patent application 140,556, and Hoekema A (1985) Ph.D. Thesis, Rijksuniversiteit Leiden, The Netherlands, disclose micro-Ti plasmids carrying ocs genes constructed from pTi15955. Bevan M (1984) Nucl. Acids Res. 12:8711-8721, discloses a kanamycin-resistant micro-Ti. T-DNA need not be on a plasmid to transform a plant cell; chromosomally located T-DNA is functional (Hoekema A et al. (1984) EMBO J. 3:2485-2490). T-DNA has direct repeats of about 25 base pairs associated with the left and right borders, i.e. with the T-DNA/plant DNA junctions, which may be involved in either transfer from Agrobacterium or integration into the host genome. Ti plasmid-determined characteristics have been reviewed by Merlo, supra (see especially Table II therein), and Ream and Gordon, supra .

Transformation-Inducing Plasmid DNA

Different octopine-type Ti plasmids are nearly 100% homologous to each other when examined by DNA hybridization or restriction enzyme analysis.

A portion of the Ti or Ri plasmid is found in the DNA of tumorous plant cells. T-DNA is often integrated (i.e. inserted) into host DNA at multiple sites in the nucleus. Flanking plant DNA may be either repeated or low copy number sequences. Integrated T-DNA can be found in either direct or inverted tandem arrays and can be separated by spacers. T-DNA can also transform chloroplasts (De Block M et al. (1985) EMBO J. 4:1367-1372).

Octopine-type Ti plasmids integrate two separate T-DNAs, $T_L$-DNA and $T_R$-DNA, left and right T-DNAs, respectively. Though T-DNA is occasionally deleted after integration in the plant genome, it is generally stable.

The exact location relative to the border repeats of T-DNA/flanking plant DNA junctions varies and need not be within a border repeat. Virulence is not always eliminated after deletion of one of either of the usual nopaline T-DNA border sequences (e.g., see Hepburn AG and White J (1985) Plant Mol. Biol. 5:3-11). Sequences flanking a border repeat can affect transformation efficiency (Peralta EG and Ream LW (1985) Proc. Natl. Acad. Sci. USA 82:512-5116). The orientation of the right nopaline border can be reversed without total loss of functionality, and a single border sequence is capable of transforming closely-linked sequences (De Block M et al. (1984) EMBO J. 3:1681-1689). A synthetic 25 bp nopaline right border repeat is functional (Wang K et al. (1984) Cell 38:455-462). Circular intermediates associated with T-DNA transfer appear to be spliced precisely within the 25 bp direct repeats (Koukolikova-Nicola Z et al. (1985) Nature 313:191-196).

Shuttle Vectors

Shuttle vectors have proven useful for manipulation of Agrobacterium plasmids. They were developed by Ruvkun GB and Ausubel FM (1981) Nature 289:85-88, and provide means for inserting foreign genetic material into large DNA molecules, include copies of recipient genome DNA sequences into which the foreign genetic material is inserted. Shuttle vectors can be introduced into the ultimate recipient cells, by well known methods, including the tri-parental mating technique (Ruvkin and Ausubel, supra), direct transfer of a self-mobilizable vector in a bi-parental mating, direct uptake of exogenous DNA by Agrobacterium cells ("transformation"), spheroplast fusion of Agrobacterium with another bacterial cell, or uptake of liposome-encapsulated DNA. After the shuttle vector is introduced into the recipient cell, possible events include a double cross-over with one recombinational event on either side of the marker (homogenotization). Phenotypically dominant traits may be introduced by single cross-over events (cointegration) (Caplan A et al. (1983) Science 222:815-821; Horsch RB et al. (1984) Science 223:496-498); one must guard against deletion of the resulting tandem duplication.

Foreign Gene Expression

Many genes, usually non-dicot genes, have not been expressed in transformed plant cells under control of their own promoters (e.g. rabbit β-globin, yeast alchohol dehydrogenase (Adh), Adh I and ribulose-1,5-bis-phosphate carboxylase (RuBP-Case) large subunit from maize, soybean leghemoglobin, interferon and globin from mammals, and the mammalian virus SV40). However, many plant genes have been transcribed, and even translated, after T-DNA-mediated transformation (e.g. genes for nopaline synthase, bean phaseolin, Zea mays zein, RuBP-Case small subunit, wheat chlorophyll a/b binding protein, and a soybean heat shock protein).

Selectable or Screenable Markers

The nos promoter can drive expression of drug resistance structural genes useful for selection of transformed plant cells. Bevan MW et al. (1983) Nature 304:184-187; Fraley RT et al. (1983) Proc. Natl. Acad. Sci. USA 80:4803-4807; and Herrera-Estrella L et al. (1983) EMBO J. 2:987-995, inserted the bacterial kanamycin resistance structural gene (neomycin phosphotransferase II, NPT2), or kan, from Tn5 downstream from (i.e. behind or under control of) the nopaline synthase promoter. The constructions were used to transform plant cells which in culture were resistant to kanamycin and its analogs such as neomycin and G418. Promoters for octopine $T_L$ genes ORF24 and ORF25 could also drive structural gene expression (Velten J et al. (1984) EMBO J. 3:2723-2730; Gelvin SB et al. (1985) Mol. Gen. Genet. 199:240-248). Herrera-Estrella et al., supra, reported a similar construction, in which a methotrexate resistence gene - (dihydrofolate reductase, DHFR) from Tn7 was placed behind the nos promoter. Transformed cells were resistant to methotrexate. Furthermore, Herrera-Estrella L et al. (1983), Nature 303:209-213, have obtained expression in plant cells of enzymatic activity of octopine synthase and bacterial chloramphenicol acetyltransferase (CAT) by placing their structural genes under control of nos promoters. Helmer G et al. (1984) Biotechnol. 2 :520-527, have created a fusion gene having the promoter and 5'-end of the structural gene of nos fused to E. coli β-galactosidase (lacZ) sequences. Plant tissues transformed with this screenable marker may be recognized by a characteristic color when grown on the appropriate chromogenic substrate. The nos /structural gene combinations are inherited from transformed plants in a Mendelian manner (Horsch RB et al. (1984) Science 223:496-498; De Block M et al . (1984) EMBO J. 3:1681-1689).

A chimeric RuBP-Case small subunit/NPT2 protein was translocated into the chloroplast (Van den Broeck G et al. (1985) Nature 313 :358-363). That gene's promoter confers light-inducible expression in tobacco or soybean callus to CAT or NPT2 structural genes (Herrera-Estrella L et al. (1984) Nature 310:115-120; Facciotti D et al. (1985) Biotechnol. 3 :241-246).

The NPT2 gene under control of a cauliflower mosaic virus (CaMV) promoter was expressed in plant cells transformed by T-DNA (Koziel MG et al. (1984) J. Mol. Appl. Genet. 2:549-562). In work utilizing CaMV as a vector, a DHFR gene from R67 behind the CaMV 35S promoter similarly conferred methotrexate resistance to infected turnip plants (Brisson N et al. (1984) Nature 310:511-514). Tobacco mosaic virus - (TMV) coat protein has been expressed in transformed tobacco tissue under control of a CaMV promoter (Bevan MW et al. (1985) EMBO J. 4:1921-1926).

SUMMARY OF THE INVENTION

One object of this invention is to provide means for expression of structural genes within plant cells wherein said genes are foreign to said cells. Another object is to provide disarmed plant transformation vectors compatible with the growth of morphologically normal plants. Yet another object is to provide specialized plant tissues and plants having within them proteins encoded by foreign structural genes and, in cases where the protein is an enzyme, having or lacking metabolites or chemicals which respectively are not or are otherwise found in the cells in which the genes are inserted. Towards these ends, sub-Ti plasmids containing $T_R$-DNA are provided. Further objects and advantages will become evident from the following description.

The invention disclosed herein provides sub-Ti plasmids based on octopine pTi $T_L$-DNA, that is having borders A and B while lacking borders C and D. In the preferred embodiments these sub-Ti plasmids lack functional onc genes and include a functional ocs gene. The use of sub-Ti plasmids, as disclosed herein, containing the direct border repeats involved in incorporation of the T-DNA into the plant genome and a antibiotic-resistance-type plant-selectable marker can have the following useful results: (1) onc genes can be deleted resulting in greater success of plant regeneration from transformed tissue cultures or protoplasts. (2) Both the antibiotic-resistance-type marker (e.g. kan ) and ocs genes can serve as selectable markers. Their presence, especially that of ocs, can also be screened for. (3) Sub-Ti plasmids are relatively small, thereby facilitating or eliminating many manipulations otherwise required during the process of plant transformation. (4) Selection of transformed plant tissues with kanamycin is more efficient than selection with aminoethylcysteine (AEC), i.e. higher plant transformation rates, in terms of tranformed plant tissues obtained, are observed. (5) One may simultaneously select for resistance to two substances, AEC and an antibiotic such as kanamycin. (6) Inclusion of a drug-selectable marker increases the size of the sub-Ti plasmid's T-DNA. This results in an increased plant transformation frequency. (7) Use of the ORF1 promoter to drive expression of a plant-expressible selectable marker eliminates the need to place another promoter in front of the marker's structural gene. This reduces by one the number of promoters needed to express genes within tranformed T-DNA. Therefore a promoter that would otherwise be used as part of a selectable marker can be used to drive expression of other genes without introducing a sequence duplication in the T-DNA. (8) A unique restriction (BglII) site can be between two actively transcribed genes. Therefore an extraneous coding DNA may be insulated from the effects (e.g resulting from transcription) of flanking plant DNA. (9) By placing an extraneous coding DNA between two actively transcribed vector genes, one can monitor both flanking vector genes to show that the entire region is actively transcribed.

The plasmid and T-DNA constructions disclosed herein can be placed in four subgroups. (1) Plasmids having no plant-selectable markers, e.g. pAK-4, pAN6, pAR2, pAR-Sal. (2) Plasmids having a plant-selectable marker under control of a promoter intrinsic to the $T_L$-DNA segments which carry borders A and B (e.g. ORF1 or tml), e.g. pBE239. (3) Plasmids having a plant-selectable marker under control of a promoter extrinsic to the vector's T-DNA but not have a seperate bacterial-selectable marker between its T-DNA border repeats (e.g. ORF24 or a CaMV promoter), e.g. pCT29, pCV10, pH4-1, pH400. pH410, pH411, pH420, pH421. (4) Plasmids having both a bacterial-selectable marker and a seperate plant-selectable marker between its T-DNA border repeats, e.g. pCT29K-2, pH506, pH575, pH592, pH1592.

The invention also provides a plant comprising a genetically modified plant cell having an extraneous coding DNA introduced by such a sub-Ti plasmid. Further, the invention provides plant tissue comprising a plant cell having a genome comprising an extraneous coding DNA. Also provided are novel strains of bacteria containing and replicating T-DNA. Additionally, the invention provides novel vectors useful in efforts to transform plants. These vectors have the ability to replicate in bacteria and comprising $T_L$-DNA. They further comprise an extraneous coding gene inserted within $T_L$-DNA contained within the vector in such manner as to be expressible in a plant cell. Furthermore, strains of bacteria harboring said vectors are disclosed.

The present invention comprises extraneous coding DNA consisting of a foreign structural gene under control of a promoter and a transcript terminator, said promoter/gene/terminator combination being inserted into a cell by means of sub-Ti plasmids based on octopine-type Ti plasmid $T_L$ -DNA. More specifically, in its preferred embodiment the invention disclosed herein further comprises expression in plant cells of foreign structural genes under control of a certain T-DNA-derived, plant-expressible promoter, derived from DNA flanking ORF1 or ORF24 structural genes or the Camv 19S transcript, after introduction via T-DNA, that is to say, by inserting the foreign structural gene into T-DNA under control of the ORF1, ORF24, or CaMV promoter and ahead of an ORF24, ORF25, ORF26, or CaMV polyadenylation site and introducing the T-DNA containing the insert into a plant cell using known means. Once plant cells expressing the foreign structural gene are obtained, plant tissues and whole plants can be regenerated therefrom using methods and techniques well known in the art. The regenerated plants are then reproduced by conventional means and the introduced genes can be transferred to other strains and cultivars by conventional plant breeding techniques. The invention in principle applies to any introduction of a foreign structural gene into any plant species into which DNA can be introduced.

The invention is useful for genetically modifying bacteria, plant cells, plant tissues, and whole plants by inserting useful foreign structural genes from other species, organisms, or strains. Such useful structural genes include, but are not limited to, genes conveying identifiable phenotypes such as the following: improved tolerance to extremes of heat or cold; improved tolerance to anaerobic conditions (e.g. water-logging), drought, or osmotic stress; improved resistance or tolerance to insect (e.g. insecticidal toxins such as the Bacillus thuringiensis crystal protein), arachnid, nematode, or epiphyte pests and fungal, bacterial, or viral diseases; the production of enzymes or secondary metabolites not normally found in said tissues or plants; improved nutritional (e.g. lectins and storage proteins such as zein or phaseolin), flavor (e.g. sweet proteins such as thaumatin), or processing properties when used for fiber or human or animal food; changed morphological traits or developmental patterns (e.g. leaf hairs which protect the plant from insects, coloring which is aesthetically pleasing, changed plant growth habits, dwarf plants, reduced time needed for the plants to reach maturity, expression of a gene in a tissue or at a time that gene is not usually expressed, and the like); male sterility; improved photosynthetic efficiency (including lowered photorespiration); improved nitrogen fixation; improved uptake of nutrients; improved tolerance to herbicides (e.g. glyphosate or triazines); increased crop yield; improved competition with other plants; genetic markers novel to the genetically modified cell; and the like. Genetic markers can be used to improve germplasm identification by the presence of one or more characteristic nucleic acid sequences, proteins, gene products, or phenotypes however identified. Genetic markers can distinguish a genetically modified plant, plant cell, or bacterial cell of the present invention from plants, plant cells, or bacteria which are not so modified, to facilitate transfer of a genetically linked or cotransformed artificially introduced DNA sequence or phenotype by other (e.g. sexual) means to other genotypes, or to facilitate identification of plants protected by patents or by plant variety protection certificates. Resistance (or tolerance) in cell or tissue culture to selective agents (i.e. selectable markers) and markers that are readily identified during screening (e.g. screenable markers such as distinctive cell-surface antigens or enzymes, like $\beta$-galactosidase, that are readily recognized visually) are also useful genetic markers. The invention is exemplified by placing a structural gene, encoding neomycin phosphotransferase II (NPT2) from Tn5 and providing a phenotype of resistance to the effects of kanamycin and its analogs (a kan gene), under control of a promoter region which in nature controls expression of either the ORF1, ORF24, or CaMV 19S transcripts and ahead of a polyadenylation site found between ORF25 and ORF26 or at the end of the CaMV 19S transcript. The promoter/kan polyadenylation site combination can be used to detect and select cells transformed by the combination. Any DNA sequences linked to the combination may be selected for, both in eukaryotes and prokaryotes, and cells transformed by linked DNA sequences may therefore be identified, as will be understood by those in the art. Other uses of the invention, exploiting the properties of other structural genes introduced into various plant species, will be readily apparent to those skilled in the art.

DESCRIPTION OF THE DRAWINGS

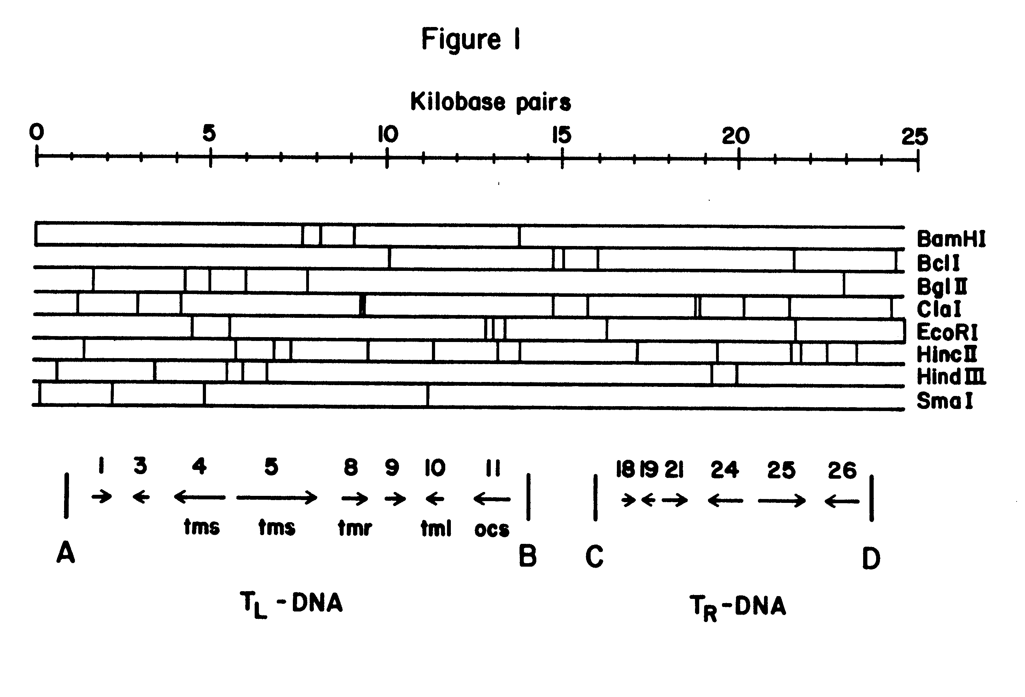

Figure 1 is a map of octopine-type T-DNA. A map of positions of restriction sites relevant to the Examples is presented below a distance scale. Positions and orientations of eukaryotic open reading frames (ORFs) are indicated by arrows. ORFs corresponding to onc genes and ocs are indicated; note that ORFs 4 and 5 are both part of tms . Positions of border repeats are indicated by vertical bars. The directions "left" and "right" in both the Description, Examples, and Claims in is as defined in this Figure. In other words, when the sequence of a T-DNA fragment is lined up with the corresponding T-DNA sequence of this Figure, the left end of the fragment is the end that, on the map, is furthest to the left

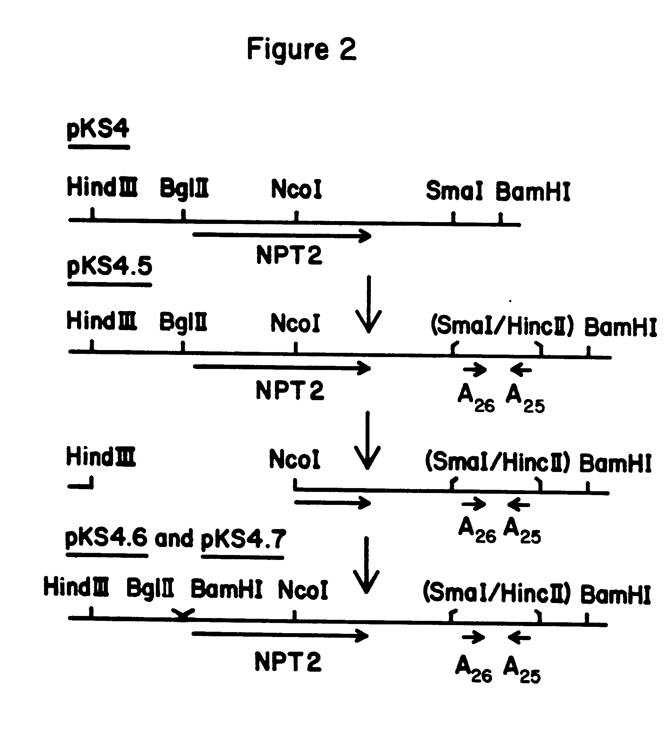

Figures 2-9 are schematic and are not drawn to scale. They use a number of common symbols. Plasmid names are underlined. Resistance genes for ampicillin, chloramphenicol, and tetracycline are respectively represented by amp, cam and tet. A suture (a join of two DNA segments resulting from ligation) of two different restriction sites which can no longer be cleaved by either enzyme is indicated parenthetically; e.g. "(SmaI/HincII)" represents a suture between a SmaI site and a HincII site which cannot be cleared by either enzyme. Similarly, a site whose specificity has been changed is indicated parenthetically; e.g. "BglII (EcoRV)" represents an BglII site in a location formerly occupied by a EcoRV site. DNA sequence position coordinates are given within square brackets. Arrows indicate locations and orientations of promoters, structural genes, polyadenylation sites, entire genes, and portions of genes. $P_1$, $P_{24}$ and $P_C$ respectively indicate the promoters of the T-DNA ORF1 gene, the T-DNA ORF24 gene, and the CaMV 19S transcript. $A_{24}$, $A_{25}$, $A_{26}$, and $A_C$ respectively indicate polyadenylation sites for the T-DNA ORFs 24, 25, and 26 and the CaMV 19S transcript. T-DNA border repeats A and B are represented by the circled A and the circled B, respectively.

Figure 2 diagrams the placement of a 0.7kb HincII fragment carrying $A_{26}$ and $A_{25}$ behind an NPT2 structural gene, followed by substitution of mutated 5'-flanking regions for the wild-type flanking regions.

Figure 3 is a diagram of pDOB513.

Figure 4 is a diagram of pMAN514.

Figure 5 is a diagram of pDOB513K4.6 and pDOB513K4.7.

Figure 6 is a diagram of pManKan-sm and pManKan-dm.

Figure 7 diagrams construction of pAN6 from p102, p233G, and pSUP106.

Figure 8 diagrams construction of a disarmed T-DNA cassette carried by pCT29K-2 from pAR-Sal, pUC4, and pDOB513K4.6. This cassette was used to make pH506, pHH575, pH592, and pH1592.

Figure 9 diagrams pH400.


DETAILED DESCRIPTION OF THE INVENTION

The following definitions are provided in order to remove ambiguities to the intent or scope of their usage in the specification and claims.

Promoter: Refers to sequences at the 5'-end of a structural gene involved in initiation of transcription. In nature the T-DNA promoter region sequences exemplified herein causes transcription in crown gall tumors of the ORF24. Eukaryotic promoter sequences are commonly recognized by the presence of DNA sequences homologous to the canonical form 5'...TATAA...3' about 10-30 base pairs (bp) 5'-to the location of the 5'-end of the mRNA (cap site). About 30 bp 5'-to the TATAA another canonical sequence is often found which is recognized by the presence of DNA sequences homologous to the canonical form 5'...CCAAT...3'. Translational initiation is usually most efficient at the first 5'...AUG...3' 3'-from the cap site.

Transcript Terminator: Refers herein to any nucleic acid sequence capable of determining the position of the 3'-end of a transcript. The transcript terminator DNA segment may itself be a composite of segments derived from a plurality of sources, naturally occurring or synthetic, prokaryotic, or eukaryotic, and may be from a genomic DNA or an mRNA-derived cDNA. Transcript termination sites include polyadenylation sites and sites determining the 3'-end of ribosomal RNAs (rRNAs), transfer RNAs, (tRNAs), and nonpolyadenylated messenger RNAs (mRNAs) (e.g. histone mRNA, P. A. Krieg and D. A. Melton (1984) Nature 308:203-206). As used herein, transcript terminators actually determine the 3'-end of the mRNA, not the end of transcription, the origonal 3'-end being removed by posttranscriptional processing (see Birnstied ML et al. - (1985) Cell 41:349-359).

Polyadenylation Site: Refers herein to a nucleic acid sequence correlated with polyadenylation of mRNA in eukaryotes, i.e. after transcriptional termination polyadenylic acid "tails" are added to the 3'-end of mRNA precursors. Polyadenylation sites are commonly recognized by the presence of homology to the canonical form 5'...AATAAA...3', although variations of distance 5' to the 3'-end of the transcript, partial "read-thru", and multiple tandem canonical sequences are not uncommon. DNA sequences between 20 and 35 bp downstream from the transcripts 3'-end seem to be necessary (M. A. McDevitt et al. (1984) Cell 37:993-999). It should be recognized that a canonical "polyadenylation site" may actually determine the location of the 3'-end of the mRNA and not polyadenylation per se (N. Proudfoot (1984) Nature 307:412-413).

Transcription controlling sequences: Refers to a promoter/polyadenylation site combination flanking a structural gene.

Foreign structural gene: As used herein includes that portion of a gene comprising a DNA segment coding for a RNA, protein, polypeptide or portion thereof, possibly including a translational start codon, foreign to, i.e. not naturally found in, $T_R$-DNA. A foreign structural gene may be derived in whole or in part from prokaryotic DNA, eukaryotic DNA, episomal DNA, plasmid DNA, plastid DNA, genomic DNA, cDNA, viral DNA, viral cDNA, chemically synthesized DNA, or the like. It is further contemplated that a foreign structural gene may contain one or more modifications in either the coding segments or untranslated regions which could affect the biological activity or chemical structure of the expression product, the rate of expression or the manner of expression control. Such modifications include, but are not limited to, mutations, insertions, deletions, and substitutions of one or more nucleotides, and "silent" modifications that do not alter the chemical structure of the expression product but which affect intercellular localization, transport, excretion or stability of the expression product. The structural gene may constitute an uninterrupted coding sequence or it may include one or more introns, bounded by the appropriate plant functional splice junctions, which may be obtained from synthetic or a naturally occurring source. The structural gene may be a composite of segments derived from a plurality of sources, naturally occurring or synthetic, coding for a composite protein, the composite protein being foreign to the cell into which the gene is introduced and expressed or being derived in part from a foreign protein. The foreign structural gene may be a fusion protein, and in particular, may be fused to all or part of a $T_R$-DNA structural gene derived from that which the transcriptional conrol sequences were derived.

Extraneous coding DNA: Refers to a DNA segment which, when expressed in a plant cell, conveys a useful or identifiable phenotype. Such phenotypes include those suggested for foreign structural genes in the Summary. An extraneous coding DNA usually will include a DNA sequence which is to be expressed as an RNA flanked by a promoter and a transcript terminator. In many cases the expressed RNA will be a translatable messenger RNA. An extraneous coding DNA may comprise a foreign structural gene undercontrol of a T-DNA promoter (e.g. the ORF1 promoter) that is an extrinsic component of the sub-Ti plasmid.

Replicon : Refers herein to a fundamental unit of replication comprising all the genetic elements sufficient to confer autonomous replication in a bacterial cell, e.g. Agrobacterium. Individual replicons differ in the extent to which they are functional in different host cell species. Many of the replicons commonly employed in plasmids for genetic engineering are functional only in bacteria of the E. coli group. Others are able to replicate in a wide range of gram-negative bacterial hosts, including Agrobacterium. A replicon will always include an origon of replication. A replicon will also include genes for functions necessary for DNA replication in a particular host cell which are not supplied by that host cell.

Plant tissue: Includes differentiated and undifferentiated tissues of plants including but not limited to roots, shoots, pollen, seeds, tumor tissue, such as crown galls, and various forms of aggregations of plant cells in culture, such as embryos and calluses. The plant tissue may be in planta or in organ, tissue, or cell culture.

Plant cell: As used herein includes plant cells in planta and plant cells and protoplasts in culture.

Bacterial cell: As used herein includes bacteria in culture, including but not limited to biologically pure cultures, and dispersed in the environment.

Positions in, open reading frames (ORFs) of, orientations (e.g. "left" and "right", both as directions and as ends of a DNA fragment or segment) of, and border sequences of octopine T-DNA are defined as numbered or designated by R. F. Barker et al. (1983) Plant Mol. Biol. 2:335-350. CaMV positions are defined by Franck A et al. (1980) Cell 21:285-294. pBR322 positions are as defined by Sutcliffe JG (1979) Cold Spring Harbor Symp. Quant. Biol. 43:79-90. Tn5 positions are as defined by Beck E et al. (1982) Gene 19:327-336.

Production of a genetically modified cell transformed by a $T_L$ -DNA-derived sub-Ti plasmid and expressing an extraneous coding DNA combines the specific teachings of the present disclosure with a variety of techniques and expedients known in the art. In most instances, alternative expedients exist for each stage of the overall process. Alternative expedients include, but are not limited to the particular vir-gene-containing helper strain to be used as a sub-Ti plasmid helper, the particular promoter, foreign structural gene, or transcript terminator which make up the extraneous coding DNA, and the plant species to be modified and the desired regeneration strategy, all of which present alternative process steps which those of ordinary skill are able to select and use to achieve a desired result. It will be understood that there may be minor sequence variations within DNA sequences utilized or disclosed in the present application. These variations may be determined by standard techniques to enable those of ordinary skill in the art to manipulate and bring into utility the promoter regions of such homologous genes. As strains harboring novel helper plasmids are developed for use in binary plant transformation systems, those of ordinary skill in the art will be able to select among those alternative process steps to achieve a desired result. The fundamental aspects of the invention are the nature of the $T_L$-DNA-derived sub-Ti plasmid and its use to transform plant cells with an extraneous coding DNA. Other aspects include the nature and structure of the foreign structural gene and the promoter controlling structural gene expression in plant cells. The remaining steps of the preferred embodiment for obtaining a genetically modified plant include inserting the promoter/structural gene/transcript terminator combination into $T_L$ -DNA-derived sub-Ti plasmid, transferring the sub-Ti plasmid to an Agrobacterium helper cell and then transforming a plant cell wherein the $T_R$-DNA becomes stably integrated as part of the plant cell genome, techniques for in vitro culture and eventual regeneration into whole plants, which may include steps for selecting and detecting transformed plant cells and steps of transferring the introduced T-DNA from the originally transformed strain into commercially acceptable cultivars, and monitoring expression in transformed plants.

A principal feature of the present invention in its preferred embodiment is the construction of a T-DNA derivative having a promoter and a transcript terminator combined with foreign structural gene, as these terms have been defined, supra, to form an extraneous coding DNA. The structural gene must be inserted in correct position and orientation with respect to both the promoter and the transcript terminator. It is known that eukaryotic promoters and transcript terminators promote and terminate transcription in one direction only along the DNA. The terminator region of DNA is said to lie "downstream" or alternatively "behind" or "3'-to" the transcribed structural gene. Similarly, the promoter is "upstream", "ahead of", or "5'-to" the structural gene. Therefore, to be functional, the correct position of promoter or the terminator must be respectively "upstream" or "downstream" from the foreign structural gene. Orientation refers to the directionality of the structural gene. That portion of a structural gene which ultimately codes for the amino terminus of the foreign protein is termed the 5'-end of the structural gene, while that end which codes for amino acids near the carboxyl end of the protein is termed the 3'-end of the structural gene. Correct orientation of the foreign structural gene is with the 5'-end thereof proximal to the promoter and the 3'-end thereof proximal to the transcript terminator.

Another principal feature of the present invention in its preferred embodiments is use of plasmids comprising modified $T_L$ -DNA, into which a promoter structural gene/transcript terminator combination is inserted, said plasmids being capable of independent replication in an Agrobacterium strain. As reviewed in the Background, border-delimited T-DNA of such plasmids can be transferred from an Agrobacterium strain to a plant cell provided the Agrobacterium strain contains certain trans-acting genes whose function is to promote the transfer of T-DNA to a plant cell. Borders are polar sequences, being nonselfcomplementary. Pairs of borders can function together when present in parallel orientation, i.e. when having homologous sequences on the same DNA strand. Comparison of published border sequences gives the consensus sequence 5'TGGAGGATAT $\begin{smallmatrix}T&C&A&G&T\\A&G&G&A&G\end{smallmatrix}$ GCTAA $\begin{smallmatrix}A&T\\T&C\end{smallmatrix}$ 3', the 5'-end and the 3'-end being respectively towards the left and right ends of both nopaline and octopine T-DNA, as conventionally mapped in the literature (e.g. as in Fig. 2). It is known in the art that the borders can be sufficient for transfer function but that sequences which flank the borders can affect transformation efficiency. Plasmids that contain T-DNA bounded by borders and which are able to replicate independently in an Agrobacterium strain are herein termed sub-Ti plasmids. Sub-Ti plasmids may have borders derived from Ti plasmids, Ri plasmids, or synthetic DNA. A spectrum of variations is possible in which the sub-Ti plasmids differ in the amount of T-DNA they contain. One end of the spectrum retains all of the T-DNA from a transformation inducing plasmid, and is termed herein mini-Ti plasmid. At the other end of the spectrum, all but an amount of DNA surrounding the T-DNA borders is deleted, the remaining portions being the minimum necessary for the sub-Ti plasmid to be transferrable and integratable in the host cell. Such plasmids are termed herein micro-Ti. Sub-Ti plasmids are advantageous in that they can be small and relatively easy to manipulate directly, eliminating the need to transfer the gene to T-DNA from a shuttle vector by homologous recombination. After the desired structural gene has been inserted, they can easily be introduced directly into an A. tumefaciens or A . rhizogenes cell containing the trans-acting vir genes that promote T-DNA transfer. Introduction into an Agrobacterium strain is conveniently accomplished either by transformation of the Agrobacterium strain or by conjugal transfer from a donor bacterial cell, the techniques for which are well known to those of ordinary skill. The present invention includes sub-Ti plasmids based on $T_L$-DNA, that is having borders A and A and lacking borders C and D. The $T_L$-DNA-derived sub-Ti plasmids disclosed herein are advantageous in that they lack all onc genes and are therefore disarmed. They are also advantageous in that they have ocs genes usefull for screening or selecting transformed plant cells.

As will be apparent to those of ordinary skill in the art, the extraneous coding DNA may be placed between any restriction sites convenient for removing the combination from the plasmid it is carried on and convenient for insertion into the plant transformation or shuttle vector of choice. Location of an extraneous coding DNA insertion site within a plant transformation vector is not critical as long as the transfer function of sequences immediately surrounding the T-DNA borders are not disrupted, since in prior art studies these regions appear to be essential for insertion of the modified T-DNA into the plant genome. Also, the coding DNA should not disrupt the function of any selectable or screenable markers needed for identification of transformed plant cells. The T-DNA into which the combination is inserted is obtained from any of the octopine-type Ti plasmid having a restriction map essentially identical to pTi15955, and the coding DNA is inserted by standard techniques well known to those skilled in the art. The orientation of the extraneous coding DNA with respect to the direction of transcription and translation of endogenous T-DNA or vector genes is not critical, either of the two possible orientations is functional, unless, of course, its expression is being controlled by a promoter intrinsic to the sub-Ti plasmid (e.g. the ORF1 promoter). Differences in rates of expression in plants may be observed when a given combination is inserted at different locations or orientations within T-DNA.

Following the strategy just described, the modified T-DNA can be transferred to plant cells by any technique known in the art, e.g. by direct infection of plants, by infection of leaf disks, or by cocultivation of plant cells with the novel Agrobacterium strain containing a foreign structural gene incorporated within T-DNA (see Background). Transformed. cells must be selected or screened to distinguish them from untransformed cells. Selection is most readily accomplished by providing a selectable marker incorporated into the T-DNA (see Background). As exemplified herein, the promoter/structural gene/transcript terminator is a selectable marker, an ORF1, ORF24, or CaMV 19S promoter/NPT2 structural gene/ORF24, ORF25, ORF26, or CaMV polyadenylation site combination, suitable for selection of transformed plant tissues. Screening methods well known to those skilled in the art include, but are not limited to specific hybridization to characteristic nucleic acid sequences, or immunological assays. Additionally, a phenotype of any expressed gene located between the T-DNA borders can be used to identify transformed tissue.

Regeneration of transformed cells and tissues is accomplished by resort to known techniques. An object of the regeneration step is to obtain a whole plant that grows and reproduces normally but which retains integrated T-DNA. The techniques of regeneration vary somewhat according to principles known in the art, depending upon the origin of the T-DNA, the nature of any modifications thereto and the species of the transformed plant. The herein disclosed Sub-Ti plasmids based on octopine-type $T_L$-DNA have no functional onc genes, are therefore totally disarmed, and can be use to create transformed tissues which may be regenerated by protocols directly derived from protocols developed for regeneration of untransformed tissues. The transformed tissue regeneration protocols generally will not need undue experimentation for adaption from untransformed tissue regeneration protocols, the changes resulting primarily from plant tissue infection steps and not from presense of the $T_L$-DNA vector in the transformed tissue.

The genotype of the plant tissue transformed is often chosen for the ease with which its cells can be grown and regenerated in in vitro culture and for susceptibility to the selective agent to be used. Should a cultivar of agronomic interest be unsuitable for these manipulations, a more amenable variety is first transformed. After regeneration, the newly introduced extraneous coding DNA/$T_L$-DNA-derived sub-Ti plasmid combination may be readily transferred to the desired agronomic cultivar by techniques well known to those skilled in the arts of plant breeding and plant genetics. Sexual crosses of transformed plants with the agronomic cultivars yield initial hybrids. These hybrids can then be back-crossed with plants of the desired genetic background. Progeny are continuously screened and/or selected for the continued presence of integrated foreign DNA or for a new phenotype resulting from expression of genes carried by the inserted foreign DNA. In this manner, after a number of rounds of back-crossing and selection, plants can be produced having a genotype essentially identical to the agronomically desired parents with the addition of inserted foreign DNA sequences.


EXAMPLES

The following Examples are presented for the purpose of illustrating specific embodiments within the scope of the present invention without limiting the scope; the scope being defined by the Claims. Numerous variations will be readily apparent to those of ordinary skill in the art.

The Examples utilize many techniques well known and accessible to those skilled in the arts of molecular biology and manipulation of transformation inducing plasmids and Agrobacterium; such methods are fully described in one or more of the cited references if not described in detail herein. Enzymes are obtained from commercial sources and are used according to the vendor's recommendations or other variations known to the art. Reagents, buffers and culture conditions are also known to those in the art. Reference works containing such standard techniques include the following: R. Wu, ed. (1979) Meth. Enzymol. 68, R. Wu et al ., eds. (1983) Meth. Enzymol. 100 and 101, L. Grossman and K. Moldave, eds. - (1980) Meth. Enzymol. 65, J. H. Miller (1972) Experiments in Molecular Genetics, R. Davis et al. (1980) Advanced Bacterial Genetics , R. F. Schleif and P. C. Wensink (1982) Practical Methods in Molecular Biology, and T. Maniatis et al. (1982) Molecular Cloning. Additionally, R. F. Lathe et al. (1983) Genet. Engin. 4:1-56, make useful comments on DNA manipulations.

Textual use of the name of a restriction endonuclease in isolation, e.g. "BclI", refers to use of that enzyme in an enzymatic digestion, except in a diagram where it can refer to the site of a sequence susceptible to action of that enzyme, e.g. a restriction site. In the text, restriction sites are indicated by the additional use of the word "site", e.g. "BclI site". The additional use of the word "fragment", e.g. "BclI fragment", indicates a linear double-stranded DNA molecule having ends generated by action of the named enzyme (e.g. a restriction fragment). A phrase such as "BclI/SmaI fragment" indicates that the restriction fragment was generated by the action of two different enzymes, here Bcl I and SmaI, the two ends resulting from the action of different enzymes. Note that the ends will have the characteristics of being "sticky" (i.e. having a single-stranded protrusion capable of base-pairing with a complementary single-stranded oligonucleotide) or "blunt" and that the specificity of a sticky-end will be determined by the specificity of the enzyme which produces it.

Positions and open reading frames (ORFs) of octopine-type T-DNA are as defined for pTi15955 by R. F. Barker et al. (1983) Plant Mol. Biol. 2:335-350, and are diagramed in Fig. 1.

Generally, plasmids, and only plasmids, are prefaced with a "p", e.g., pTi15955 or pBR322. Strain designations parenthetically indicate a plasmid harbored within, e.g., A. tumefaciens (pTi15955). The following strains are on deposit:

E. coli K802 (pH4-1) NRRL B-18009

E. coli K802 (pCT29K-2) NRRL B-18010

E. coli C600 (pKS4) NRRL B-15394
A. tumefaciens (pTi15955) ATCC 15955
In general, other plasmids and strains are widely available and accessible to those in the art.

Example 1: Preparation of an NPT2 Structural Gene

The kanamycin resistance gene (kan) of Tn5 encodes the enzyme neomycin phosphotransferase II (NPT2). The kan gene has been sequenced (Beck E et al . (1982) Gene 19:327-336) and may be extracted from pKS4, a pBR322 derivative. pKS4 DNA may be isolated from E. coli C600 (pKS4) which is on deposit as NRRL B-15394.

Although the Tn5 kan structural gene has proven useful for construction of plant-expressible selectable markers, its unmodified sequence is not optimum for eukaryotic selection. Usually eukaryotic translation is preferentially initiated at the first AUG of an mRNA; optimum initiation efficiency occurs if there is an A or G at position -3 and G at position +4 relative to the translational start site. (The A of the AUG is +1 and the base pair immediately before that A is -1.) In other words, 5' $\overset{A}{G}$ NNAUGG3' is a consensus sequence for optimum eukaryotic translational initiation sites (Kozak M (1983) Microbiol. Rev. 47:1-45; see also Rogers SG et al. (1985) Plant Mol. Biol. Reptr. 3 :111-116. In suboptimum cases, initiation can also occur at the second AUG. Commonly, extraction of the kan structural gene from its vector involves the restriction enzyme BglII, a Bgl II site being about 30 bp upstream from the kan translational start site (AUG). Between the BglII site and the translational start is an AUG triplet sequence in a different reading frame than that encoding NPT2. Also, the translational start for the kan gene does not match the consensus sequence. To remedy these deficiencies in the Tn5 kan gene sequence, two mutant sequences were constructed. One mutantation introduced a Bam HI site between the translational start and the upstream ATG, and is herein referred to as the "single mutant". The other mutantion changed a C to an A at position -3 relative to the AUG translational start in addition to introducing a BamHI site, and is herein referred to as the "double mutant". The wild-type (wt), single mutant (sm), and double mutant (dm) sequences are compared below. Features described on the top line are indicated by underlining. Introduced single base pair substitutions are indicated by arrows below the sequence.

```
        5'  BglII                1st  BamHI      2nd        3'

wt:   ...AGATCTGATCAAGAGAACAGGATGAGGATCGTTTCGCATG...kan...


sm:   ...AGATCTGATCAAGAGAACAGGATGAGGATCCTTTCGCATG...kan...
                                            ↑

dm:   ...AGATCTGATCAAGAGAACAGGATGAGGATCCTTTAGCATG...kan...
                                            ↑    ↑
```

Procedures for chemical synthesis and manipulation of DNA oligonucleotides utilize techniques well known to those skilled in the art of DNA synthesis. Oligonucleotide-directed, site-specific mutagenesis has been reviewed recently by Craik CS (1985) Biotechniques 3:12-19; Zoller MJ and Smith M (1983) Meth. Enzymol. 100:468-500; Smith M and Gillam S (1981) in Genetic Engineering: Principals and Methods, Vol. 3, eds.: Setlow JK and Hollaender A; and Smith M (1982) Trends Biochem. Sci. 7:440-442. This technique permits the change of one or more base pairs in a DNA sequence or the introduction of small insertions or deletions. Recent examples of oligonucleotide-directed mutagenesis include Carter P et al. (1985) Nucl. Acids Res. 13:4431-4443; Zoller MJ and Smith M (1984) DNA 3:479-488; Kramer W et al. (1984) Nucl. Acids Res. 12:9441-9456; Zoller and Smith (1983) supra; Zoller MJ and Smith M (1982) Nucl. Acids Res. 10:6487-6500; Dalbadie-McFarland G et al. (1982) Proc. Natl. Acad. Sci. USA 79:6409-6413; and Simons GFM et al. (1982) Nucl. Acids Res. 10:821-832. Oligonucleotide-directed mutation using double-stranded DNA vectors is also possible (Wallace RB et al. (1980) Science 209:1396-1400; Vlasuk GP et al. (1983) J. Biol. Chem. 258:7141-7148; Lewis ED et al. (1983) Proc. Natl. Acad. Sci. USA 80 :7065-7069; Morinaga Y et al. (1984) Biotechnol. 2:636-639; Schold M et al . (1984) DNA 3:469-477). Useful M13-based vectors include mWB2341 and related vectors (Barnes WM (1983) Meth. Enzymol. 101:98-122; Barnes WM and Bevan M - (1983) Nucl. Acids Res. 11 :349-368), and the M13mp-series of vectors (e.g. see J. Norrander et al. (1983) Gene 26:101-106, Messing J and Vieira J (1982) Gene 19:269-276)

pKS4 DNA was digested with Smal to open it at the Tn 5-derived Smal site. After this linearized DNA was mixed with and ligated to ClaI linkers, the DNA transformed into E. coli K802. Plasmid DNAs isolated from transformants resistant to ampicillin and kanamycin were screened by restriction mapping and a colony was identified which harbored a plasmid, designated pKS4.2, having a kan gene that could be extracted on a ClaI fragment.

pKS4.2 was digested with ClaI and a fragment carrying the kan gene was electrophoretically isolated. This fragment was mixed with and ligated to ClaI-linearized pBR322 and transformed into E. coli. Plasmid DNAs isolated from transformants resistant to ampicillin and kanamycin were screened by restriction analysis and a colony was identified which harbored a plasmid designated pKS4.3. The pKS4.3 kan gene was oriented with its 5'-end and 3'-end respectively proximal to the pBR322 EcoRI and BamHI sites. In this orientation the kan gene may be removed on a HindIII fragment.

pKS4.3 DNA was digested with HindIII and mixed with and ligated to HindIII-linearized M13mp8 RF DNA (Vieira J and Messing J (1982) Gene 19:259-268). The ligation mixture was transformed into E. coli JM107 (Yanisch-Perron C et al. (1985) Gene 33:103-119) and, after isolation of RF DNA from transformants resistant to ampicillin and kanamycin, a colony was identified which harbored a M13-derivative designated M13HS-Kan. M13HS-Kan had the kan gene inserted into the M13mp8 HindIII site parallel to the M13mp8 lac gene. When in viral form, the NPT2 sequence was equivalent to an NPT2-encoding mRNA. In other words, the M23HS-Kan single-stranded viral DNA could not hybridize to NPT2 mRNA but was complementary to a cDNA made from NPT2 mRNA.

Single-stranded viral DNA was mixed with and hybridized to a primer having the sequence (a) 5'CAATCATGCGAAAGGATCC3'. (The underlined base indicates the position of the introduced mutation.) After primer extension, this DNA was transformed into E . coli JM107. RF DNAs isolated from transformed plaques were screened by hybridization under stringent conditions to [32]P-end-labeled primer and by restriction analysis. A colony was identified which harbored a M13HS-Kan derivative designated M13HS-Kan1 (sm). M13HS-Kan1 had a BamHI site at about -10 relative to the ATG translational start codon. Cutting at this BamHI site removes a Tn5 ATG located approximately at position -15.

The above described steps were repeated substituting a primer of sequence (b) 5'CAATCATGCT AAAGGATCC3' for primer (a). A colony was identified which harbored an M13HS-Kan-derivative designated M13HS-Kan2 (dm). M13HS-Kan2 had a BamHI site at about -10 relative to the translational start codon and a substitution of an A for a C at -3.


Example 2: Combination of the NPT2 Genes with a T-DNA Polyadenylation Site

The transcript terminators of pTi15955 ORFs 25 and 26 are both located within a DNA segment bounded by HincII sites at positions 21,727 and 22,440, as defined by Barker et al., supra (see Fig. 1). This 714 bp HincII fragment carries polyadenylation sites of two antiparallel genes, ORFs 25 and 26. When this HincII fragment, or any DNA segment having two antiparallel polyadenylation sites, is placed behind a structural gene, one or the other polyadenylation sites will be functional for promoting transcript termination in a plant cell. This HincII fragment is a subfragment of octopine pTi EcoRI fragment 22.

Some alternative blunt-ended pTi15955-derived segments carrying antiparallel transcript terminators, the restriction enzyme(s) which generate them, and their sizes are as follows:

| ORFs | Enzyme(s) | Size (bp) |
|------|-----------|-----------|
| 9/10 | HaeI | 1034 |
| 9/10 | HaeIII | 915 |
| 9/10 | NaeI/SmaI | 807 |
| 21/24 | AluI | 755 |
| 21/24 | ThaI | 960 |
| 25/26 | HaeI | 1163 |
| 25/26 | PvuII/StuI | 1036 |

Nonblunt-ended segments can be obtained and either used directly or have sticky-ends converted to blunt ends by methods well known to the art; e.g. a 1.11 kbp BbvI/ClaI fragment carries terminators of pTi15955 T-DNA ORFs 1 and 3 and a 1.46 kbp NciI fragment carries terminators of ORFs 9 and 10.

A pBR322 clone of the Eco RI fragment spanning T-DNA positions 16,202 to 21,631 was digested with HincII. A 714 bp HincII fragment carrying the ORFs 25 and 26 polyadenylation sites was electrophoretically purified. pKS4 DNA digested with SmaI was mixed with and ligated to the 714 bp HincIII fragment. E. coli cells were transformed by the ligation mixture. Plasmid DNAs isolated from transformants resistant to kanamycin and ampicillin were screened by restriction analysis. A colony was identified which harbored a plasmid, designated pKS4.5, that had the 714 bp T-DNA fragment inserted into the SmaI site present in the Tn5 sequences of pKS4 3'-from the NPT2-encoding sequence (Fig. 2).

pKS4.5 DNA was digested with HindIII and NcoI. This operation deleted the 5'-end of the NPT2 gene along with some 5'-flanking sequences. The Hin dIII/NcoI-digested DNA was mixed with and ligated to M13HS-Kan1 RF DNA which had been digested with HindIII and NcoI. After transformation of the ligation mix into E . coli and selection for resistance to ampicillin, plasmid DNAs were isolated and screened by restriction analysis. A colony was identified which harbored a plasmid, designated pKS4.6, which was essentially identical to pKS4.5 except for a single-base substitution which created a BamHI site just 5'-to the NPT2 encoding sequence (Fig. 2.).

pKS4.5 DNA digested with HindIII and Nco I was mixed with and ligated to M13HS-Kan2 RF DNA which had been digested with HindIII and NcoI. After transformation, selection, isolation of plasmids, and restriction analysis, a colony was identified which harbored a plasmid, designated pKS4.7, which was essentially identical to pKS4.5 except for two single-base substitutions. One substitution created a BamHI site just 5'-to the NPT2 encoding sequence, as was done with pKS4.6, while the other changed a C to an A at position -3 to the NPT2 translational start (Fig. 2).


Example 3: Preparation of CaMV Transcription Controlling Sequences

pDOB512, carrying cauliflower mosaic virus (CaMV) transcription controlling sequences (obtained from Dr. Ken Richards, Centre National de la Recherche Scientifique, Institute de Biologie Moleculaire et Cellulaire, 15, rue Descartes, F-67084 Strasbourg, France) was constructed as follows: (For a review of CaMV, see Hohn T et al. (1982) Curr. Top. Microbiol. Immunol. 96:193-236.) A HindIII fragment carrying the CaMV 19S RNA promoter region (CaMV nucleotides 5376-5851) was inserted into pBR322 and was trimmed back to within one base pair of the 19S transcript cap site. An adapter molecule having both a SmaI site and a BamHI (the structure being 5'CCGGGGGATCCGG3': 5'CCGGATCCCCGGG3', see below) was then ligated to the trimmed DNA. A HincII fragment carrying the CaMV 19S transcript terminator (CaMV nucleotides 7018-7794) to which BamHI linkers had been added was then inserted behind the 19S promoter, the promoter and terminator being separated by the SmaI/BamHI linker. The resulting plasmid is designated pDOB412. pDOB412 DNA was digested with BglII and SalI, filled in by incubation with the Klenow fragment of E. coli DNA polymerase I, and religated, thereby deleting DNA, which includes BamHI and HindIII sites, between the CaMV position 7644 BglII site and the pBR322 position 650 SalI site and regenerating a BglII site. The resultant plasmid was designated pDOB512.

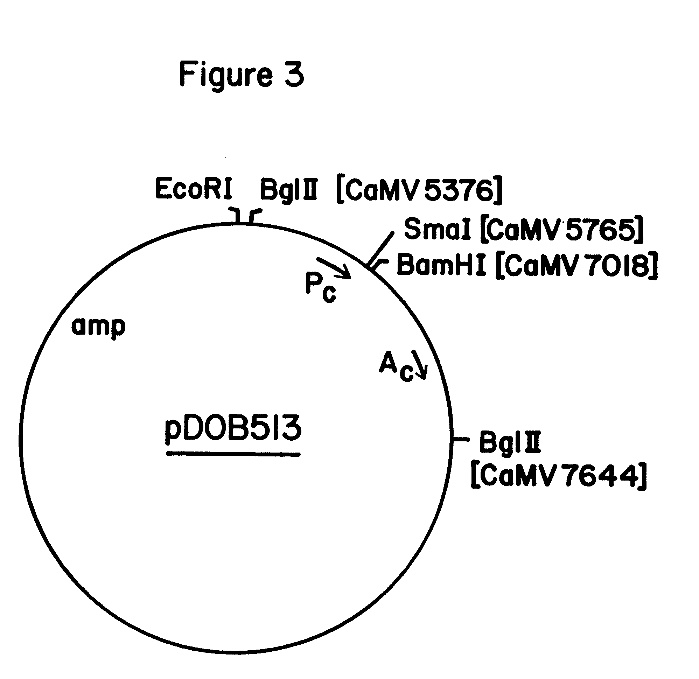

The sticky-ends of HindIII-linearized pDOB512 DNA were converted to blunt-ends. The blunt-ended pDOB512 DNA was mixed with and ligated to commercially available BglII linkers. The ligation mix was transformed into E. coli K802 and an ampicillin-resistant transformant was isolated which harbored a plasmid, designated pDOB513 (Fig. 3). pDOB513 has CaMV 19S transcription controlling sequences on a BglII fragment. SmaI and BamHI sites are found between the DNA segments having the promoter and the polyadenylation site in both pDOB412, pDOB512, and pDOB513, thereby providing a convenient location for insertion of foreign DNA that is to be a template for a transcript. The sequence of this suture is as follows:


<pre>
            SmaI   BamHI
    5'...GAGAAAATCAGCCCGGGGATCCGGAACA...3'
</pre>


The first underlined base, an A, corresponds to the first base of the 19S mRNA, transcription being towards the right, and the second and third underlined bases, respectively a G and an A, are derived from CaMV sequences at positions 5765 and 7018, respectively, as defined by Hohn et al., supra.


Example 4: Preparation of T-DNA ORF24 Transcription Controlling Sequences

pKS111 is a recombinant plasmid having Ti15955 T-DNA which spans the sequence between the Eco RI sites at positions 16,202 and 21,631 inserted into the Eco RI site of pRK290. E. coli K802 methylates DNA at many, but not all, ClaI sites, thereby specifically protecting certain sites from the action of ClaI. The only susceptible ClaI sites of pKS111 are the sites at T-DNA positions 18,892 and 20,128. These sites define a DNA fragment which includes the ORF24 structural gene but does not include the ORF24 promoter or polyadenylation site. ORF24 is associated with mannopine synthesis. Plasmid DNA isolated from E. coli K802 (pKS111) was digested with ClaI, ligated to itself, and transformed into K802. A tetracycline-resistant transformant was identified by restriction analysis which harbored a plasmid identical to pKS111 except for deletion of a fragment which spanned the ClaI sites at positions 18,892 and 20,128.

pBR322 has a BamHI site which will prove inconvenient for later manipulations. Therefore, the T-DNA of the Cla I-deleted pKS111-derivative was transferred to a pBR325 derivative which was lacking a BamHI site. pBR325 DNA, isolated from E. coli GM33 (pBR325), was digested with both BamHI and BclI, was ligated to itself and was transformed into E. coli. Plasmid DNA isolated from transformants sensitive to tetracycline and resistant to chloramphenicol and ampicillin were characterized by restriction mapping and a colony was identified which harbored a plasmid, designated pBR325aBB, which could not be cleaved with either BamHI or BclI. DNA of the ClaI-deleted pKS111-derivative was digested with Eco RI and was mixed with and ligated to EcoRI-linearized, dephosphorylated pBR325aBB DNA. After transformation into E. coli, plasmid DNAs isolated from transformants resistant to ampicillin and sensitive to both chloramphenicol and tetracycline were characterized by restriction mapping. A colony was identified which harbored a plasmid having the ClaI-deleted T-DNA recombined with the pBR325aBB vector.

Plasmid DNA from this transformant was isolated, and cleaved at its single ClaI site (the suture between the sites at positions 18,892 and 20,128) by ClaI. After the ClaI sticky-ends were then removed by incubation with the Klenow fragment of E. coli DNA polymerase I, the blunt-ended DNA was mixed with and ligated to commercially available BamHI linkers. After digestion of the BamHI-linkered DNA with BamHI, religation to itself, and transformation of the resulting DNA into K802, plasmid DNAs were isolated from transformants resistant to ampicillin were screened by restriction mapping. A colony was identified which harbored a plasmid, designated p403BRL1, lacking a ClaI site at the T-DNA positions 18,892/20,128 suture but, instead, having a BamHI site at the former location of that ClaI site.

pDOB513 DNA was digested with BglII, religated to itself, and transformed into K802. Colonies which harbored a plasmid, designated pDOB514, having no CaMV sequences were identified by restriction mapping of the harbored plasmids.

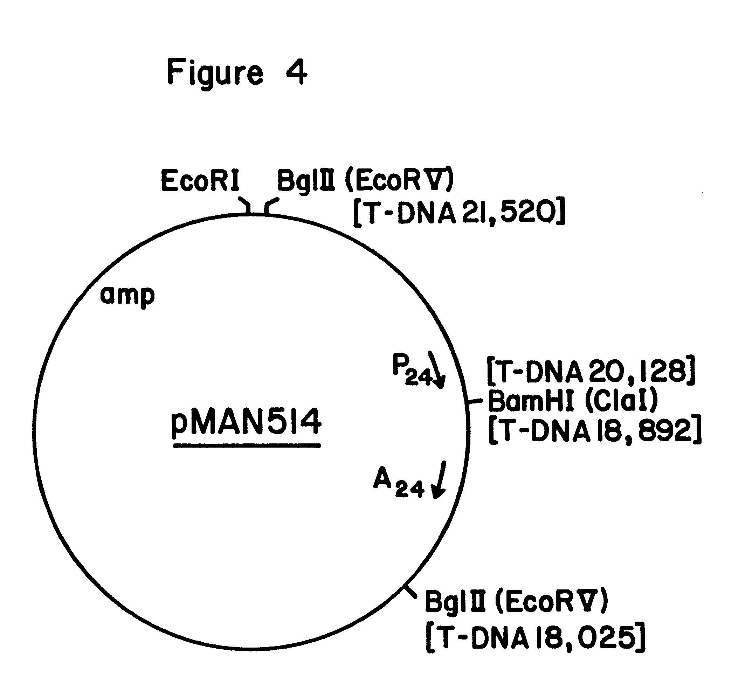

p403BRL1 DNA, which had a BamHI site between the ORF24 promoter, and transcript terminator, was then digested with EcoRV, which cleaves at sites which in T-DNA correspond to positions 18,027 and 21,522. The restriction digested DNA had its sticky-ends removed by incubation with the Klenow fragment of E. coli DNA polymerase I. The blunt-ended DNA was then mixed with and ligated to commercially available BglII linkers, digested with BglII, and subjected to agarose gel electrophoresis. A 2.26 kbp fragment was mixed with and ligated to BglII-linearized pDOB514 DNA. Plasmid DNAs of ampicillin-resistant transformants were characterized by restriction mapping, and a colony was identified which harbored a plasmid, designated pMAN514 (Fig. 4), having a 2.29 kbp BglII fragment carrying a ORF24 promoter and polyadenylation site separated by a BamHI site.


Example 5: Combination of an NPT2 Structural Gene with Plant Transcription Controlling Sequences

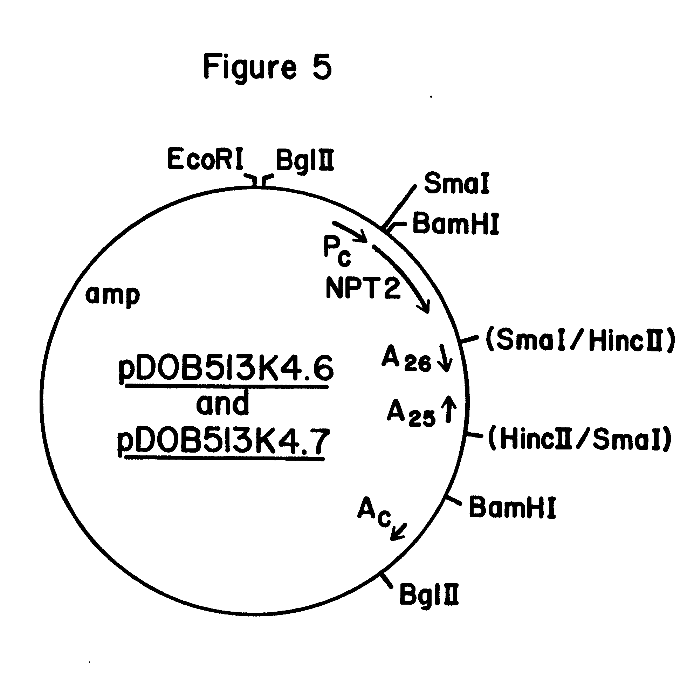

The NPT2 structural gene was removed from pKS4.6 (single-mutant: BamHI site present but the C at -3 not changed to an A) by digestion with BamHI. After the 2.23 bp fragment was electrophoretically isolated, it was mixed with and ligated to BamHI-linearized, dephosphorylated pDOB513 DNA. The ligation mixture was transformed into K802. Plasmid DNAs isolated from ampicillin-resistant transformants were screened by restriction mapping and a colony was identified which harbored a plasmid designated pDOB513K4.6 (Fig. 5). The NPT2 structural gene of this plasmid was oriented with its 5'-end proximal and its 3'-end distal to the CaMV 19S promoter.

15

The above described procedures were repeated, substituting pKS4.7 (double mutant: BamH5 site present and the C at -3 changed to an A) for pKS4.6 (single mutant: BamHI site present but -3 unchanged). A colony was identified that harbored a plasmid, designated pDOB513K4.7 (Fig. 5), which was essentially identical to pDOB513K4.6 except for a substitution of an A for a C 3 bp 5' to the translational start of the NPT2 structural gene.

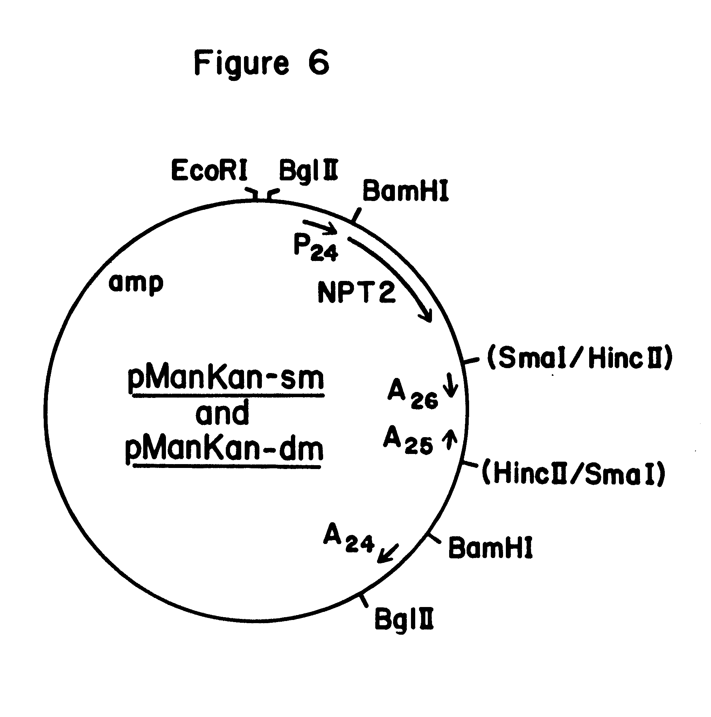

pKS4.6 DNA digested with BamHI was mixed with and ligated to BamHI-linearized pMAN514 DNA. After transformation of E. coli and selection for resistance to ampicillin, plasmid DNAs were isolated and restriction mapped. A colony was identified which harbored a plasmid, designated pManKan-sm (Fig. 6), which had the BamHI fragment carrying both the NPT2 structural gene and ORF26 polyadenylation site inserted into the BamHI site of pMan514. The NPT2/ORF26 fragment was oriented with the 5'-end of the kan gene proximal to the ORF24 promoter and the ORF26 polyadenylation site proximal to the ORF24 polyadenylation site. The orientation was such that a plant RNA polymerase II initiating transcription off of the ORF24 promoter would transcribe an mRNA encoding NPT2 before reaching the ORF26 and ORF24 polyadenylation sites, the ORF26 site being 5'-to the ORF24 site.

The above described procedures were repeated substituting pKS4.7 (double mutant) for pKS4.6 (single mutant). A colony was identified that harbored a plasmid, designated pManKan-dm (Fig. 6), which was essentially identical to pManKan-sm except for substitution of an A for a C at position -3 relative to the NPT2 translational start site.


Example 6: Construction of the Micro-Ti Plasmid pAN6

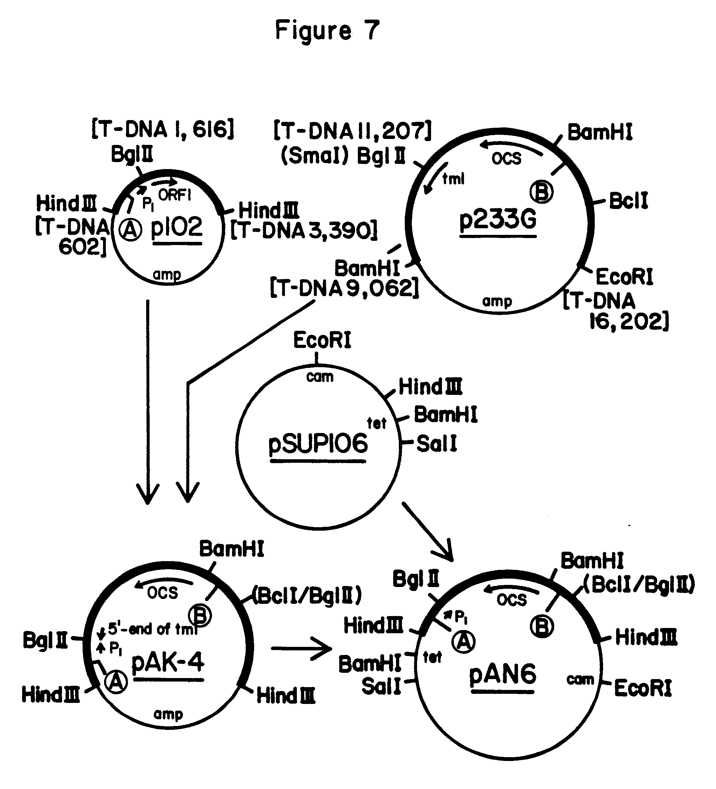

p102 (Fig. 7), a pBR322 clone of the pTi15955 T-DNA fragment between HindIII sites at positions 602 and 3,390 (as defined by Barker RF et al. (1983) Plant Mol. Biol. 2:335-350) carries the left border of $T_L$ and promoter sequences associated with ORF1. p233 is a pBR322 clone of the pTi15955 T-DNA BamHI/EcoRI fragment spanning positions 9,062 and 16,202. The T-DNA of p233 includes a Sma I/BclI fragment spanning positions 11,207 and 14,711 having ocs, a 3'-deleted tml, and the right border of $T_L$. p233 was linearized with Sma I, mixed with and ligated to a commercially available blunt-end BglII linker, trimmed with BglII, religated to itself , and transformed into E. coli GM33 (a dam ⁻ host that does not methylate DNA in a manner incompatible with the action of BclI, Marinus MG and Morris NR (1974) J. Mol. Biol. 85:309-322). A colony was identified which harbored a plasmid, designated p233G (Fig. 7), having a BglII site in the location formerly occupied by the position 11,207 SmaI site.

p233G DNA was digested with BglII and Bcl I and a 3.5 kbp fragment was isolated by agarose gel electrophoresis followed by elution. The 3.5 kbp BglII/BclI fragment was mixed with and ligated to BglII-digested, dephosphorylated p102 DNA. The ligation mixture was transformed into E . coli K802 (Wood WB - (1966) J. Mol. Biol. 16 :118). Plasmids DNAs from ampicillin-resistant transformants were characterized by restriction analysis and a colony was identified, designated pAK-4 (Fig. 7), having the BglII/BclI fragment of p233G inserted into the BglII site of p102 and oriented so that the ocs gene was located between the left and right $T_L$ borders. One BglII site, also between the borders, was regenerated, and a BglII/BclI suture, not susceptible to the action of either enzyme, was generated to the right of the right border. pAK-4 may be represented as follows:

.... pBR322 ... HindIII ... left border ... ORF1 promoter ...BglII ... 5'-end of tml ... ocs ... right border ... ( BglII/BclI) ... HindIII ... pBR322 ....

The T-DNA of pAK-4 may be removed on a 6 kbp Hin dIII fragment. HindIII-digested pAK-4 DNA was mixed with and ligated to HindIII-linearized, dephosphorylated pSUP106 DNA. pSUP106, a 11 kbp wide host-range plasmid capable of maintenance in both E. coli and Agrobacterium (Priefer UB et al. (1985) J. Bacteriol. 163:324-330), is harbored by E. coli CSH52 (pSUP106) which is on deposit as NRRL B-15486. The reaction mixture was transformed into K802 and plasmid DNAs from chloramphenicol-resistant transformants were characterized by restriction analysis. A colony was identified harboring a plasmid, designated pAN6 (Fig. 7), having the Agrobacterium DNA of pAK-4 inserted into the HindIII site of pSUP106 oriented so that BglII/BclI suture was proximal to the pSUP106 EcoRI site. pAN6 is a micro-Ti plasmid having within its two T-DNA borders a functional ocs gene and a BglII site that is unique to the plasmid. The BglII site is flanked by an incomplete tml gene and the ORF1 promoter, both of which are transcribed towards the BglII site.

Example 7: Construction of a micro-Ti T-DNA Cassette, part of pCT29K-2

p102 is a plasmid having T-DNA spanning the HindIII sites at T-DNA positions 602 and 3,390 inserted into the HindIII site of pBR322. T-DNA near position 602 is proximal to the pBR322 EcoRI site while T-DNA near position 3,390 is proximal to the pBR322 Sall and BamHI sites. The 3.5 kbp Bcl I/BglII fragment of p233G, which carries border repeat B, ocs, and the 5'-end of tml, was mixed with and ligated to p102 DNA that had been dephosphorylated after digestion with BamHI and BglII. The ligation mixture was transformed into E. coli. Plasmid DNAs isolated from ampicillin-resistant transformants were screened by restriction analysis and a colony was identified which harbored a plasmid designated pAR2. pAR2 has the following structure:

.... amp ... EcoRI ... pBR322 position 29 HindIII pTi15955 position 602 ... T-DNA position 1,617 BglII T-DNA position 11,207 ... T-DNA position 14,711 (BclI/BamHI) pBR322 position 375 ... pBR322 position 651 Sall ... ori ... amp ...

In other words, two pTi15955 fragments were inserted between the HindIII and BamHI sites of pBR322, a HindIII/BglII fragment spanning T-DNA positions 602 to 1,617 and a BglII/BclI fragment spanning T-DNA positions 11,207 to 14,711 (the BglII site having been converted from a Smal site at position 11,207), the two T-DNA fragments being ligated together at their BglII-derived ends to form a BglII-cleavable suture. These two T-DNA fragments were inserted so that the HindIII-end of the T-DNA was joined to the pBR322 position 29 HindIII site, thereby forming a Hin dIII-cleavable suture, and the BclI-end of the T-DNA was joined to the pBR322 position 375 BamHI site, thereby forming a suture that could not be cut by either BamHI or Bcl I.

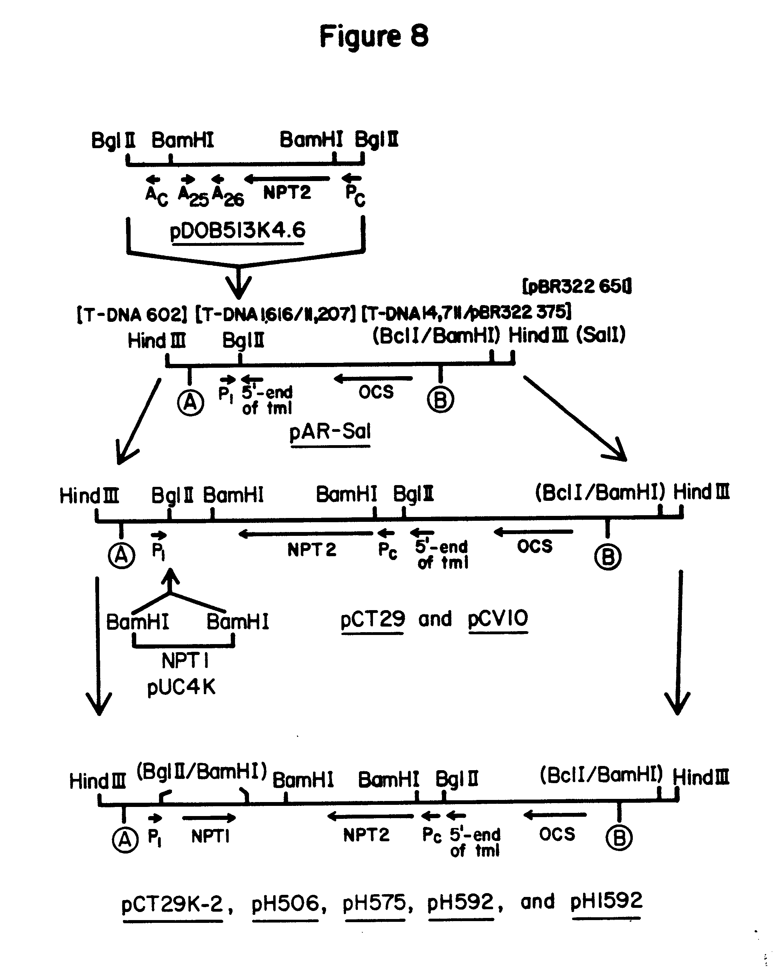

pAR2 was digested with Sall, thereby being linearized at the pBR322 position 651 Sall site. Sticky-ends of the linearized DNA were converted to blunt-ends by incubation with the Klenow fragment of DNA polymerase I, this DNA was mixed with and ligated to commercially available HindIII linkers. After partial digestion with HindIII and religation of the mixture to itself, the mixture was transformed into E. coli. Plasmid DNAs isolated from ampicillin-resistant transformants were screened by restriction analysis and a colony was identified which harbored a plasmid designated pAR-Sal (Fig. 8). pAR-Sal was identical to pAR2 except for conversion of the pAR2 Sall site to the specificity of HindIII (one or more HindIII sites may be present at this location). A 4.8 kbp HindIII fragment could be isolated from pAR-Sal which carried the T-DNA of a micro-Ti, having between Borders A and B the ORF1 promoter, a unique BglII site, the 5'-end of a tml gene, and a functional ocs gene.

pDOB513K4.6 DNA was digested with BglII and a 3.26 kbp fragment carrying the CaMV 19S promoter/NPT2 structural gene/CaMV and T-DNA polyadenylation sites combination was electrophoretically isolated. This BglII fragment was mixed with and ligated to BglII-linearized, dephosphorylated pAR-Sal DNA. The ligation mixture was transformed into E. coli. Plasmid DNAs isolated from ampicillin resistant transformants were restriction mapped and a colony was isolated which harbored a plasmid, designated pCT-29 - (Fig 8), that had the promoter/NPT2 gene/polyadenylation site combination inserted into the BglII site of pAR-Sal. The combination was oriented so that it was transcribed parallel to the 5'-end of tml and the ocs gene and antiparallel to the ORF1 promoter. pCT-29 had two Bgl II sites

pCT-29 DNA was digested with HindIII. A 8.0 kbp fragment carrying the T-DNA of pCT-29 was mixed with and ligated to HindIII-linearized, dephosphorylated pRK292 DNA. The ligation mixture was transformed into E. coli. Plasmids DNAs isolated from transformants resistant to tetracycline and sensitive to ampicillin were restriction mapped and a colony was identified which harbored a plasmid, designated pCV10 (Fig. 8), the T-DNA-bearing HindIII fragment of pCT-29 inserted into the HindIII site of pRK292. pCV10 has two BglII sites. It may serve as a micro-Ti plasmid.

Kanamycin resistant is a useful genetic marker to use when transforming Agrobacterium. As the CaMV promoter/NPT2 structural gene combination is not expressed in bacteria, it was useful to include a kan gene in the micro-Ti cassette. A neomycin phosphotransferase I (NPT1) gene from Tn903 is present on pUC4K (Vieira J and Messing J (1982) Gene 19:259-268). pUC4K DNA was digested with BamHI and a 1.45 kbp fragment carrying an NPT1 gene was electrophoretically isolated. This BamHI fragment was mixed with and ligated to linear, dephosphorylated pCT-29 DNA that had been linearized with BglII under partial digestion conditions. The ligation mixture was transformed into E. coli. Restriction mapping of plasmid DNAs led to identification of an ampicillin-resistant, kanamycin-resistant transformant which harbored a plasmid designated pCT29K-2 (Fig. 8). pCT29K-2 has an NPT1 gene that is expressible in bacteria inserted into the BglII site found between the ORF1 promoter and the NPTII gene. The BglII/BamHI sutures between the pUC4K fragment and the pCT-29 vector could not be cleaved by either BglII or BamHI; pCT29K-2 had only one BglII site, located between the NPT2 and ocs genes. The NPT1 gene was oriented parallel to the ORF1 promoter and antiparallel to the NPT2 gene, the 5'-end of tml, and the ocs gene. The T-DNA can be

17

represented as follows: borderA ... bacteria selectable NPT1 ... unique BglII site ... plant selectable NPT2 ... 5'-end of tml ... ocs ... border B. This T-DNA can be removed from pCT29K-2 on a 9.52 kbp HindIII fragment.

Example 8: A pTi Having a disarmed T-DNA

Triparental matings

Triparental matings were generally accomplished as described below; other variations known to those skilled in the art are also acceptable. pRK290 and pRK2013 were disclosed by G. Ditta et al. (1980) Proc. Natl. Acad. Sci. USA 77 :7347-7351, and pPH1J1 by P. R. Hirsh (1978) Thesis, Univ. E. Anglia. E. coli RR1 (pRK290-based-shuttle-vector) or E. coli K802 (pRK290-based-shuttle-vector) was mated with E. coli RR1 - (pRK2013) and A348, a transformation-inducing plasmid harboring A. tumefaciens strain resistant to rifampicin. After pRK2013 was transferred to the shuttle vector carrying strain, pRK2013 mobilized the pRK290-based shuttle vector for transfer to the Agrobacterium. Growth on a minimal medium incapable of supporting the growth of E. coli , AB glucose, containing both rifampicin and the drug to which the shuttle vector is resistant, often either kanamycin or carbenicillin (an ampicillin analog), resulted in the selection of Agrobacterium cells containing pRK290-based shuttle vector sequences. A mating of these cells with E. coli (pPH1J1), strain 2104, resulted in the transfer of pPH1J1 to the Agrobacterium cells. pPH1J1 and pRK290-based shuttle vectors cannot coexist for long in the same cell. Growth on gentamycin and kanamycin select for cells which have Ti plasmids that have undergone single-or double-homologous recombination events (cointegration or homogenotization, respectively) with the shuttle vector and now carry the desired construction.

pH1592

pH592 was a pRK290-based shuttle vector. (pRK292 was a pRK290 derivative having the pRK290 BglII site converted to a HindIII site by insertion of a linker.) pH592 was mated into A. tumefaciens 15955 (which is available to the public as ATCC 15955) by the triparental mating technique (see below). A colony was identified by restriction mapping and selection which was the result of double homologous recombination event. This colony harbored a plasmid, designated pH1592 (Fig. 8), which was essentially identical to pTi15955 with the exception of substitution of the NPT1 bacterial selectable marker and the NPT2 plant selectable marker for pTi15955 sequences between the BglII and SmaI sites at T-DNA positions 1,617 and 11,207, respectively. This substitution disarmed pH1592, as no onc genes remain in $T_L$ and none are known to be present in $T_R$.

Example 9: Construction of Micro-Ti Plasmids Having Selectable Markers

pH4-1

pDOB513K4.6 DNA was digested with BglII and a 3.26 kbp fragment carrying the CaMV 19S promoter/NPT2 structural gene/CaMV and T-DNA polyadenylation sites combination was electrophoretically isolated. This BglII fragment was mixed with and ligated to BglII-linearized, dephosphorylated pAN6 DNA. The ligation mixture was transformed in E. coli. Plasmid DNAs isolated from transformants resistant to chloramphenicol were screened by restriction mapping and a colony was identified which harbored a plasmid, designated pH4-1, that had the promoter/NPT2 gene/polyadenylation site combination inserted into the BglII site of pAN6. The combination was oriented so it was transcribed parallel to the tml and the ocs promoters and antiparallel to the ORF1 promoter.

pH400

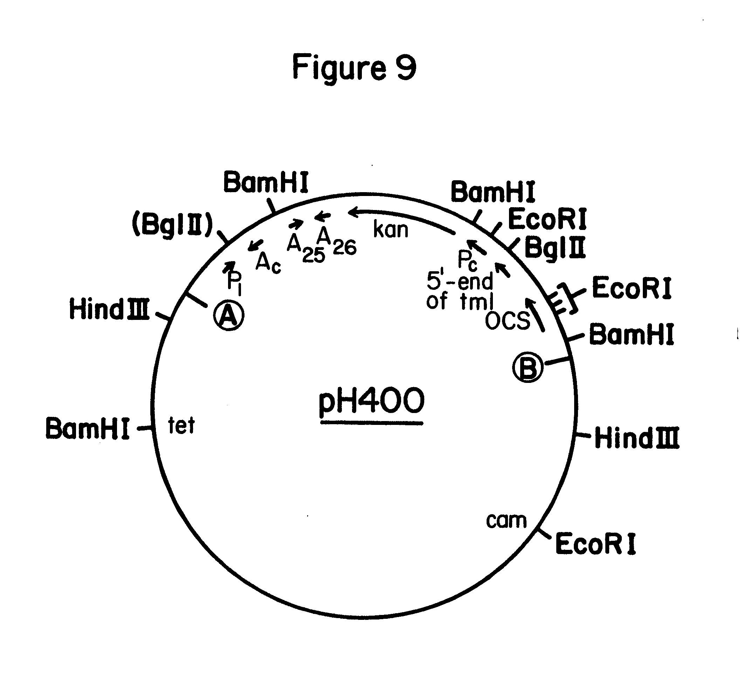

pH4-1 has two BglII sites, both of which flank the kan selectable marker. One of the BglII sites was removed, thereby leaving a unique BglII site useful for insertion of extraneous coding DNA. pH4-1 DNA was linearized by being partially digested with BglII and full-length, linear DNA was electrophoretically isolated. The Bgl II sticky-ends were then removed by incubation with the Klenow fragment of E. coli DNA polymerase I. The resulting blunt-ended DNA was ligated to itself and transformed into E. coli. Plasmid DNAs isolated from transformants resistant to chloramphenicol were screened by restriction analysis and a colony was identified which harbored a plasmid designated pH400 (Fig. 9). pH400 is identical to pH4-1 except for the absense of the BglII site between the kan gene and the ORF1 promoter, the unique pH400 BglII site being located between the kan gene and the ocs gene. The single BglII site is a convenient place to insert extraneous coding DNA, in particular if that DNA has compatble 5'GATC...3' sticky-ends resulting from the action of Bgl II, BclI, BamHI, MboI, or Sau3AI. Alternatively, the BglII site may be converted to that of another restriction enzyme, provided that pH400 does not already have a site for that enzyme. Furthermore, the sticky-ends can be converted to blunt ends and a blunt-ended DNA fragment can be blunt-end ligated into the gap, as is understood in the art.

pH506, pH575, and pH592

The micro T-DNA-carrying 9.52 kbp HindIII fragment of pCT29K-2 was mixed with and ligated to various HindII-linearized, dephosphorylated vector DNAs. The vectors were pSUP106 (Priefer et al., supra), pTJS75 (see Klee HJ et al. (1985) Biotechnol. 3:637-642), and pRK292 (Ditta G et al. (1985) Plasmid 13:149-153). Restriction mapping of E. coli transformants resistant both to kanamycin and to either chloramphenicol, tetracycline, or tetracycline (resistance genes respectively contributed by pSUP106, pTJS75, or pRK292) resulted in identification of colonies harboring plasmids designated pH506, pH575, and pH592 (Fig. 8). These plasmids are derivatives, respectively, of pSUP106, pTJS75, and pRK292.

pBE239

After pKS4.5 DNA was digested with BglII and BamHI, it was mixed with and ligated to BglII-linearized, dephosphorylated pAN6 DNA. After transformation into E. coli, plasmid DNAs isolated from chloramphenicol-resistant transformants were screened by restriction analysis. A colony was identified which harbored a plasmid designated pBE239. pBE239 has a NPT2 structural gene/ORF26 polyadenylation site combination inserted into the pAN6 BglII in such orientation that a NPT2-encoding mRNA may be transcribed, transcripts being initiated by pAN6's ORF1 promoter and being terminated by the ORF26 polyadenylation site.

pH410, pH420, pH411, and pH421

pH410 was constructed essentially as described for pH4-1, except that pManKan-sm was substituted for pDOB513K4.6. Therefore, pH410 was essentially the same as pH4-1 except that the NPT2 structural gene was transcribed off of a ORF24 promoter rather than a CaMV promoter.

The geneology of pH420 was essentially the same as that of pH410 except that the source of the NPT2 structural gene was the pKS4.7 doubly mutated kan gene rather than the singly mutated pKS4.6 gene. In other words, the two plasmids differ in sequence essentially only at position -3 from the NPT2 ATG translational start, pH410 having a C (wild-type) and pH420 having an A (mutated).

pH411 and pH421 were constructed essentially as described for pH410 and pH420, respectively, except that the ORF24 promoter/NPT2 structural gene/polyadenylation site combinations were inverted. The pH410 and pH420 kan genes are transcribed parallel to, i.e. off of the same DNA strand as, the ocs gene. The pH411 and pH421 kan genes are transcribed antiparallel to, i.e. off the opposite DNA strand as, the ocs gene.

Example 10: Plant Transformation

Micro-Ti plasmids were transferred into A. tumefaciens LBA4404 (Ooms G et al. (1981) Gene 14:33-50), a vir gene-bearing, micro-Ti-mobilizing strain, by the triparental mating technique. Tobacco leaf tissue was inoculated using a modification of the method of Horsch RB et al. (1985) Science 227:1229-1231. Leaf segments were dipped in the innoculating suspension of bacteria for 10-30 min. The inoculating bacterial suspensions had titres of $10^7$-$10^8$ ml$^{-1}$; the exact concentration was found to be unimportant. Plates having feeder cells were not used; rather innoculated leaf segments were placed on a cell-free medium, the segments being separated from direct contact with the medium by filter paper. The medium was a tobacco regenerating medium having MS salts, a mixture well known to the art, supplemented with 0.1 mg/l p-chlorophenoxy-acetic acid (pPCPA), 7.5 mg/ml 6-($\gamma,\gamma$-dimethylallylamino)-purine (2iP), 125 mg/l each of cefoxitin and mefoxin, and 300 mg/l kanamycin. The number of individual shoots and shoot cultures were totaled, divided by the number of leaf pieces, and multiplied by 100; i.e. scores are numbers of shoots or shoot clusters per 100 pieces. Experiments were scored after 3 to 5 weeks. Generally 75-80% of the shoots were able to root in a kanamycin-containing medium and all were transformed. Some kanamycin-resistant shoots did not make measurable quantities of octopine.

| Experiment | Plasmid | Shoots (clusters)/ 100 segments |
|---|---|---|
| 1 | pH410 | 85 |
| 1 | pH421 | 57 |
| 1 | pH420 | 181 |
| 1 | pH421 | 120 |
| 1 | pH575 | 101 |
| 2 | pH506 | 4 |
| 2 | pH575 | 120 |
| 2 | pH592 | 96 |
| 3 | pH4-1 | 20 |
| 3 | pH4-1-Bt | 187 |

Experiment 1 indicated that the efficiency of transformation is affected by the orientation of the selectable marker, kan genes parallel to ocs gene being more efficient than those that are antiparallel. Experiment 1 also indicated that changing the C to an A at -3 from the NPT2 translational start more than doubled the combined transformation and selection efficiency of these micro-Ti plasmids. This experiment further indicated that the caMV 19S promoter may be more efficient than the ORF 24 promoter, having higher transformation rates than the equivalent pH410 and pH411 NPT2 genes.

Experiment 2 indicated that the replicon of a micro-Ti plasmid affects transformation efficiency, pTJS75 being slightly superior to the pRK292. (Both plasmids are based on pRK290, pTJS75 being a deleted derivative and pRK292 being identical to pRK290 except at a single restriction site.) pSUP106 was a greatly inferior replicon compared to the pRK290 derivatives.

Experiment 3 indicated that transformation efficiency can be affected by insertion of extraneous coding DNA, in this case being increased by the presence of a plant-expressible <u>Bacillus</u> <u>thuringiensis</u> crystal protein gene under control of an ORF24 promoter (pH4-1-Bt). This might be an effect of the presence of additional DNA or a result of the nature of the sequence of the particular extraneous coding DNA sequence.

Kanamycin kill curves of plant cells transformed by LBA4404 (pBE239) and T-DNAs having selectable markers using CaMV and ORF24 promoters were compared. Tissues were resistant to similar concentrations of the drug. As pBE239 has an ATG between the 5'-end of the NPT2 mRNA and the NPT2 translational start, and as Rogers SG <u>et al</u>. (1985) Plant Mol. Biol. Reptr. <u>3</u>:111-116, show that presence of that ATG lowers five-fold both the $LD_{50}$ of plant tissue to kanamycin and the assayed NPT2 activity, this result indicates that the ORF1 promoter is highly active.

All these experiments indicated that micro-Ti plasmids disclosed herein are functional and are useful for plant transformation. It will be recognized by those skilled in the art that various extraneous coding DNAs may be inserted into these vectors and subsequently transformed into plants. It will also be recognized that the present invention is not limited to the vectors disclosed herein, but that there are many possible variations within the scope of the claims that those of ordinary skill in the art may find advantageous to use.


## Claims

1. A DNA molecule comprising a first segment comprising octopine-type T-DNA sequences from a left-end at about position 909 to a right-end at about position 1,616, a second segment comprising an antibiotic resistance marker structural gene, and a third segment comprising octopine-type T-DNA sequences from a left-end at about position 12,480 to a right-end at about position 14,083, wherein the molecule lacks octopine-type T-DNA sequences from about position 1,623 to about position 11,207, and the right-end of the first segment is joined to one end of the second segment and the other end of the second segment is joined to the left-end of the third segment.

2. A DNA according to claim 1, wherein the third segment comprises octopine-type T-DNA sequences from a left-end at about position 11,203 to a right-end at about position 14,083.

3. A DNA according to claim 1 or claim 2, further comprising a replicon permitting replication in <u>Agrobacterium</u> and/or <u>E</u> . <u>coli</u>.

4. A DNA according to claim 3, wherein the DNA is selected from the group pBE239, pCT-29, pCT29K-2, pCV10, pH4-1, pH400, pH410, pH411, pH420, pH421, pH506, pH575, pH592, and Ph1592.

5. A DNA according to claim 4, wherein the DNA is pBE239.

6. A DNA according to claim 4, wherein the DNA is selected from the group pCT-29, pCV10, pH4-1, pH400, pH410, pH411, pH420, pH421.

7. A DNA according to claim 4, wherein the DNA is selected from the group pCT-29K-2, pH506, pH575, pH592 and pH1592.

8. A DNA according to claim 1, wherein the antibiotic structural gene of the second segment is in such position and orientation with respect to an ORF1 promoter present at the right end of the first segment that transcription of the structural gene in a plant cell is under control of the ORF1 promoter.

9. A DNA according to claim 8, wherein the structural gene encodes neomycin phosphotransferase.

10. A DNA according to claim 1, the second segment further comprising an antibiotic resistance marker selectable in a bacterial cell.

11. A DNA according to claim 10, wherein the bacterial selectable marker encodes neomycin phosphotransferase.

12. A DNA according to claim 1, wherein the second segment comprises a promoter, a structural gene, and a transcript terminator, the promoter and the transcript terminator having such sequence that they are functional in a plant cell, and the promoter, the structural gene, and the transcript terminator being in such position and orientation with respect to one another that transcription of the structural gene in a plant cell is under control of the promoter and the transcript terminator.

13. A DNA according to claim 12, wherein the promoter is a CaMV 19S promoter or an octopine-type T-DNA ORF24 promoter.

14. A DNA according to claim 12, wherein the transcript terminator is a polyadenylation site found behind an octopine-type T-DNA ORF24, ORF25, or ORF26 structural gene, or is a CaMV 19S transcript polyadenylation site.

15. A DNA according to claim 12, wherein the structural gene encodes neomycin phosphotransferase.

16. A DNA according to claim 15, wherein the neomycin phosphotransferase translational start is the first AUG codon in a messenger RNA transcribed in a plant.

17. A DNA according to claim 16, wherein the base three bases 5'-to the translational start is an A or a G.

18. A DNA molecule comprising CaMV 19S promoter, a structural gene encoding neomycin phosphotransferase, and an octopine-type T-DNA ORF25 or ORF26 polyadenylation site, wherein the promoter, the structural gene and the polyadenylation site are in such position and orientation with respect to each other that transcription of the structural gene in a plant cell is under control of the promoter and the polyadenylation site.

19. A DNA according to claim 18, wherein the DNA is essentially identical to the promoter/structural gene/ polyadenylation site combination of pDOB513K4.6 or pDOB513K4.7.

20. A DNA according to claim 19, wherein the DNA is pDOB513K4.6 or pDOB513K4.7.

21. A DNA according to any of claims 1 to 17, further comprising plant DNA, the plant DNA being located to the left of the first segment and to the right of the second segment.

22. A bacterial cell containing the DNA of any of claims 1 to 20.

23. A plant, plant cell, plant seed or plant tissue containing the DNA of any of claims 1 to 21.

24. A method of making a DNA molecule according to any of claims 1 to 17, comprising joining the right-end of the first segment to one end of the second segment, and the other end of the second segment to the left-end of the third segment.

25. A method of making a bacterial cell according to claim 22, comprising transforming a bacterial cell with a vector containing the DNA of any of claims 1 to 20.

26. A method of making a plant, plant cell, plant seed or plant tissue according to claim 23, said method including the step of infecting a plant with a bacterial cell according to claim 22.

Figure I

# Figure 2

# Figure 3

EcoRI  BglII  [CaMV 5376]

Sma I [CaMV 5765]

BamHI [CaMV 7018]

P$_c$

amp

A$_c$

pDOB513

BglII
[CaMV 7644]

0 223 417

# Figure 4

# Figure 5

# Figure 6

# Figure 7

# Figure 8

# Figure 9

European Patent Office

**PARTIAL EUROPEAN SEARCH REPORT**
which under Rule 45 of the European Patent Convention
shall be considered, for the purposes of subsequent
proceedings, as the European search report

Application number

## DOCUMENTS CONSIDERED TO BE RELEVANT

EP 86308064.4

| Category | Citation of document with indication, where appropriate, of relevant passages | Relevant to claim | CLASSIFICATION OF THE APPLICATION (Int. Cl.4) |
|---|---|---|---|
| A,P | EP - A2 - 0 186 425 (ELI LILLY AND COMPANY)<br><br>* Claims 1,16 *<br><br>-- | 1,12 | C 12 N 15/00<br>C 07 H 21/04<br>C 12 N 5/00<br>A 01 H 1/00<br>C 12 P 19/34<br>(C 12 N 15/00<br>C 12 R 1:19) |
| D,A | PLANT MOLECULAR BIOLOGY, vol. 2, no. 1, 1983 (The Hague)<br>R.F.BARKER et al. "Nucleotide sequence of the T-DNA region from the Agrobacterium tumefaciens octopine Ti plasmid pTi 15 955" pages 335-350<br><br>* Abstract *<br><br>-- | 1 | |
| A | WO - A1 - 84/02 919 (MONSANTO COMPANY)<br><br>* Claims 1-7 *<br><br>-- | 1 | |

**TECHNICAL FIELDS SEARCHED (Int. Cl.4)**

C 12 N
C 07 H
C 12 P
A 01 H

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: Claims 1-22,24-26
Claims searched incompletely: Claim 23 (plant seed) (Art. 53 b) EPC)
Claims not searched:
Reason for the limitation of the search:

| Place of search | Date of completion of the search | Examiner |
|---|---|---|
| VIENNA | 23-01-1986 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

| | EINSCHLÄGIGE DOKUMENTE | | KLASSIFIKATION DER ANMELDUNG (Int. Cl. 4) |
|---|---|---|---|
| Kategorie | Kennzeichnung des Dokuments mit Angabe, soweit erforderlich, der maßgeblichen Teile | betrifft Anspruch | |
| A | EP - A1 - 0 122 791 (AGRIGENETICS RESEARCH ASSOCIATES LIMITED) <br><br> * Claim 1 * <br><br> -- | 1 | |
| A | EP - A1 - 0 145 338 (AGRIGENETICS RESEARCH ASSOCIATES LIMITED) <br><br> * Claims 1,26,57,84 * <br><br> -- | 1,8,12, 18,23 | |
| D,A | EP - A1 - 0 140 556 (AGRIGENETICS RESEARCH ASSOCIATES LIMITED) <br><br> * Claims 1,7,19 * <br><br> -- | 1 | RECHERCHIERTE SACHGEBIETE (Int. Cl. 4) |
| D,A | JOURNAL OF MOLECULAR AND APPLIED GENETICS, vol. 2, no. 6, 1984 (New York) <br><br> M.G. KOZIEL et al. "A Cauliflower Mosaic Virus Promoter Directs Expression of Kanamycin Resistance in Morphogenic Transformed Plant Cells" pages 549-562 <br><br> * Totality * <br><br> -- | 18 | |
| D,A | MOLECULAR & GENERAL GENETICS, vol. 199, 1985, (Berlin, Heidelberg) <br><br> S.B.GELVIN et al. "Use of a $T_R$ T-DNA promoter to express genes in plants and bacteria" pages 240-248 <br><br> * Totality * <br><br> ---- | 1,9,11 | |